# EUROPEAN PATENT SPECIFICATION

(11) **EP 1 373 290 B1**
(45) Date of publication and mention of the grant of the patent: **20.08.2014**
(21) Application number: 02719327.5
(22) Date of filing: 21.03.2002
(51) Int. Cl.: C07H 17/08, C12P 19/62, A01N 45/02, A01N 63/00, A01N 63/02

(54) **PESTICIDAL SPINOSYN DERIVATIVES**
PESTIZIDE SPINOSYN VERBINDUNGEN
DERIVES DE SPINOSYNE PESTICIDES

(30) Priority: 21.03.2001 US 277601 P
(43) Date of publication of application: 02.01.2004
(73) Proprietor: Dow AgroSciences, LLC, Indianapolis, Indiana 46268 (US)
(72) Inventor: HAHN, Donald, R., Zionsville, IN 46077 (US); BALCER, Jesse, L., Carmel, IN 46032 (US); LEWER, Paul, Indianapolis, IN 46234 (US); GILBERT, Jeffrey, R., Indianapolis, IN 46208 (US); GRAUPNER, Paul, R., Carmel, IN 46033 (US)
(74) Representative: Beck Greener
(86) International application number: PCT/US2002/008957
(87) International publication number: WO 2002/077005

(56) References cited:
- WO-A-01/19840
- WO-A-94/20518
- WO-A-97/00265
- KIRST H A ET AL: "DISCOVERY, ISOLATION, AND STRUCTURE ELUCIDATION OF A FAMILY OF STRUCTURALLY UNIQUE, FERMENTATION-DERIVED TETRACYCLIC MACROLIDES" ACS SYMPOSIUM SERIES, WASHINGTON, DC, US, 1992, pages 214-225, XP000944494 ISSN: 0097-6156

## Description

### Field Of The Invention

This invention relates to a new group of analogs of natural pesticidal products, and to novel mutants of a *Saccharopolyspora* species that produces the compounds.

### Background Of The Invention

The naturally produced spinosyn compounds consist of a 5,6,5-tricylic ring system, fused to a 12 or 14-membered macrocyclic lactone. Spinosyns are typically substituted with a neutral sugar (rhamnose) at C-9 and a second sugar, preferably amino-substituted (e.g. forosamine) (see Kirst *et al.*, 1991; Lewer *et al.,* 2000), but also neutral (see Lewer *et al.,* 2000) at C-17. If the second sugar is not present, the compounds have been referred to as the pseudoaglycone, or if the neutral sugar (rhamnose) is not present, then the compounds have been referred to as the reverse pseudoaglycone. Two distinct classes of spinosyns are known: spinosyns and butenyl-spinosyns. These two classes of spinosyns differ in the substitution of the carbon tail attached to the macrocyclic ring at C-21. The natural spinosyns, which are disclosed in US Patent No. 5,362,634, are substituted with methyl or ethyl at C-21, while natural butenyl spinosyns, which are disclosed in WO 01/19840, are substituted at C-21 with a 3-4 carbon chain, at various levels of oxidation.

Spinosyns (A83543) are produced by derivatives of *Saccharopolyspora spinosa* NRRL18395 including strains NRRL 18537, 18538,18539, 18719, 18720, 18743 and 18823 and derivatives thereof. A more preferred nomenclature for spinosyns is to refer to the pseudoaglycones as spinosyn A 17-Psa, spinosyn D 17-Psa, etc., and to the reverse pseudoaglycones as spinosyn A 9-Psa, spinosyn D 9-Psa, etc. (see Kirst *et al.,* 1991). The known members of this family have been referred to as factors or components, and each has been given an identifying letter designation. These compounds are hereinafter referred to as spinosyn A, B, etc. The spinosyn compounds are useful for the control of arachnids, nematodes and insects, in particular *Lepidoptera* and *Diptera* species, and they are quite environmentally friendly and have an appealing toxicological profile.

U.S. Patent No. 5,362,634 and corresponding European Patent Application No. 375316 A1 disclose spinosyns A, B, C, D, E, F, G, H, and J. WO 93/09126 discloses spinosyns L, M, N, Q, R, S, and T. WO 94/20518 and US 5,6704,486 disclose spinosyns K, O, P, U, V, W, and Y, and derivatives thereof. A large number of synthetic modifications to spinosyn compounds have been made, as disclosed in U.S. Patent No. 6,001,981 and WO 97/00265.

More recently, a new family of related macrolides have been isolated from *Saccharopolyspora* sp. LW107129 (NRRL 30141) as described in WO 01/19840. Specific compounds produced by strain NRRL 30141 that have been identified to date are listed in Table I.

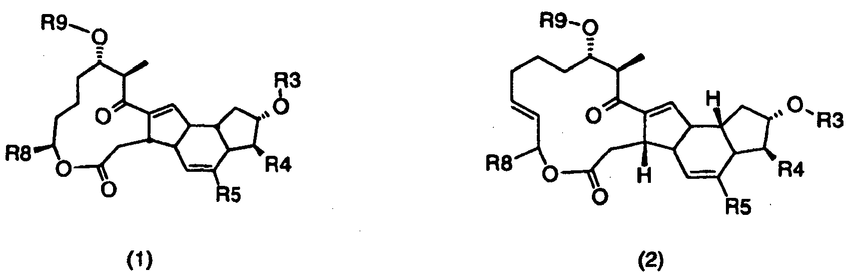

**TABLE I**

| cmpd. no. | Name | formula | R³ * | R⁴ | R⁵ | R⁸ | R⁹ ** |
|---|---|---|---|---|---|---|---|
| 1 | for-rham-I | (1) | (3a) | H | H | 1-butenyl | (9a) |
| 2 | N-oxy-for-rham-I | (1) | (3a) | H | H | 1-butenyl | (9b) |
| 3 | N-desmethyl for-rham-I | (1) | (3a) | H | H | 1-butenyl | (9c) |
| 4 | 2"-hydroxy-for-rham-I | (1) | (3a) | H | H | I-butenyl | (9d) |
| 5 | for-(2'-O-desmethyl rham)-I | (1) | (3b) | H | H | 1-butenyl | (9a) |
| 6 | for-(3'-O-desmethyl rham)-I | (1) | (3c) | H | H | 1-butenyl | (9a) |
| 7 | for-rham-II | (1) | (3a) | H | CH₃ | 1-butenyl | (9a) |
| 8 | for-rham-III | (1) | (3a) | OH | H | 1-butenyl | (9a) |
| 9 | 24-hydroxy-for-rham-I | (1) | (3a) | H | H | 3-hydmxy-1-butenyl | (9a) |
| 10 | 24-hydroxy-N-desmethylfor-rham-I | (1) | (3a) | H | H | 3-hydroxy-1-butenyl | (9c) |
| 11 | 24,25-dehydro-for-rham-I | (1) | (3a) | H | H | 1,3-butadienyl | (9a) |
| 12 | ami-rham-I | (1) | (3a) | H | H | 1-butenyl | (9e) |
| 13 | 3"-O-methyl-glu-rham-I | (1) | (3a) | H | H | 1-butenyl | (9f) |
| 14 | ami-rham-III | (1) | (3a) | OH | H | 1-butenyl | (9e) |
| 15 | mole-rham-III | (1) | (3a) | OH | H | 1-butenyl | (9g) |
| 16 | 24-demethyl-for-rham-I | (1) | (3a) | H | H | 1-propenyl | (9a) |
| 17 | rham-I | (1) | (3a) | H | H | 1-butenyl | H |
| 18 | rham-II | (1) | (3a) | H | CH₃ | 1-butenyl | H |
| 19 | rham-III | (1) | (3a) | OH | H | 1-butenyl | H |
| 20 | 24-hydroxy-rham-I | (1) | (3a) | H | H | 3-hydroxy-1-butenyl | H |
| 21 | 24-hydroxy- rham-III | (1) | (3a) | OH | H | 3-hydroxy-1-butenyl | H |
| 22 | 24,25-dehydro-rham-I | (1) | (3a) | H | H | 1,3-butadienyl | H |
| 23 | 22,23-dihydro-rham-I | (1) | (3a) | H | H | n-butyl | H |
| 24 | (4'-N-desmethyl-1",4"-diepi-for)-rham-I | (1) | (3a) | H | H | 1-butenyl | (9h) |
| 25 | 5'-epifor-rham-I | (1) | (3a) | H | H | 1-butenyl | (9i) |
| 26 | 24,25-dehydro-for-rham-III | (1) | (3a) | OH | H | 1,3-butadienyl | (9a) |
| 27 | 8-OH-for-rham II | (1) | (3a) | OH | CH₃ | 1-butenyl | (9a) |
| 28 | 24-desmethyl-for-rham-III | (1) | (3a) | OH | H | 1-propenyl | (9a) |
| 29 | 2'-O-desmethyl rham-I | (1) | (3b) | H | H | 1-butenyl | H |
| 30 | 3'-O-desmethyl rham-1 | (1) | (3c) | H | H | 1-butenyl | H |
| 31 | for-rham-IV | (2) | (3a) | H | H | ethyl | (9a) |

*R³ is a group having one of the following formulas (3a) through (3c) 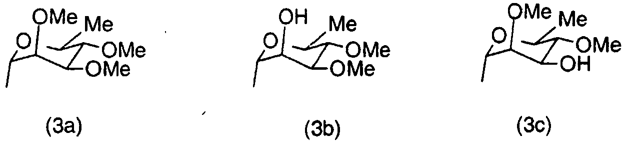 **R9 is a group having one of the following formulas (9a) to (9i)

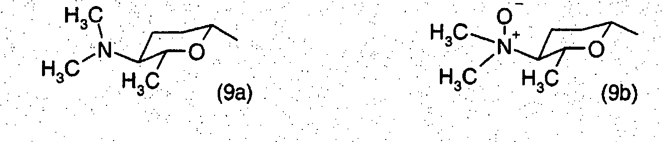

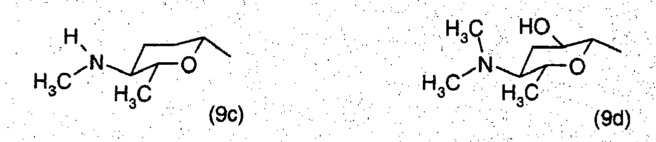

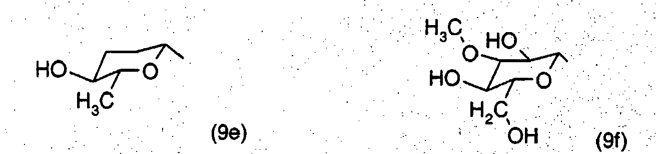

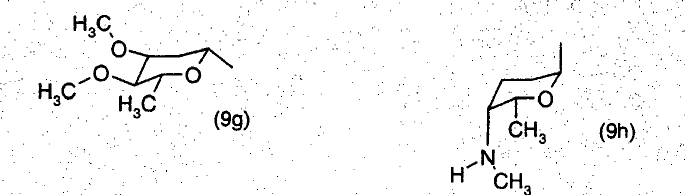

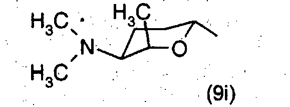

The butenyl spinosyns are named in Table I and referred to hereinafter by the structural acronyms "for-rham-I", "for-rham-II", "for-rham-III" and derivatives thereof. In these cases I, II, and III refer to the appropriately-substituted macrolide structure (I: R⁴=R⁵=H; II: R⁵ = CH₃, R⁴ = H or OH; III: R⁵ = H, R⁴ = OH), 'for' represents the sugar at C-17 (for = forosamine), and 'rham' represents the sugar at C-9 (rham = tri-*O-*methylrhamnose). A second type of macrolide structure which is produced by strain NRRL 30141, with general Formula (2) having a 14-membered macrolide ring, is referred to hereinafter as IV and the fully glycosylated comound as "for-rham-IV". The butenyl-spinosyn compounds of formulae (1) and (2) are useful for the control of arachnids, nematodes and insects, in particular *Lepidoptera* and *Diptera* species, and they are quite environmentally friendly and have an appealing toxicological profile.

The butenyl spinosyns exhibit a number of variations from the spinosyn series including extensive modifications at the C-21 position, hydroxylation at the C-8 position and substitution of alternate sugars, including neutral sugars, for forosamine at C-17. In addition, compound (31) possesses a 5,6,5-tricylic ring system, fused to a 14 membered macro-cyclic ring with forosamine and rhamnose attached at C-17 and C-9.

### SUMMARY OF THE INVENTION

The present invention provides novel isolated strains of *Saccharopolyspora* sp. designated 30141.2, 30141.3, 30141.4, 30141.5, 30141.8, and 30141.13.

The invention also provides a new series of butenyl-spinosyn compounds that can be produced by culturing these strains in suitable media, and that have the following general Formula (1A): 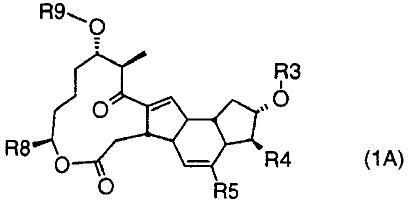 wherein:
R³ is a group selected from 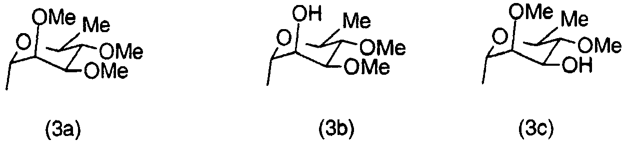 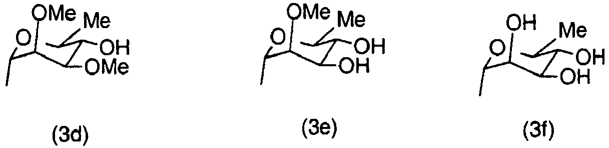
R⁴ is a hydrogen atom or a hydroxyl group,
R⁵ is a hydrogen atom or methyl group,
R⁸ is 1-butenyl, 1,3-butadienyl, n-butyl, 3-hydroxy-1-butenyl, butan-3-one, 1,2-epoxy-butyl or 1-propenyl, and
R⁹ is a hydrogen atom or a group selected from 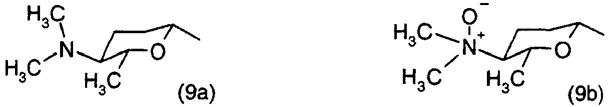 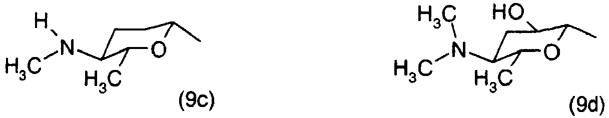 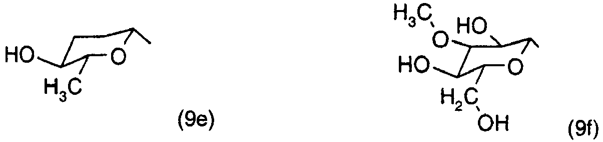 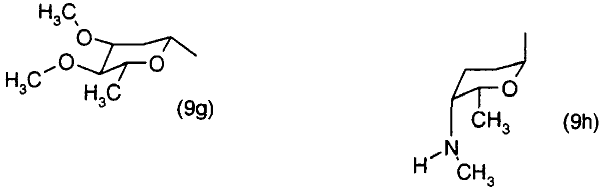 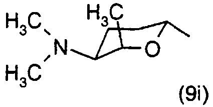 provided that:
   a) if R³ is a group of formula (3b) or (3c), R⁴ and R⁵ are H, and R⁸ is 1-butenyl, then R⁹ is neither H nor a group of formula (9a); and
   b) if R³ is a group of formula (3 a), then R⁴ and R⁵ are H, R⁸ is 1-propenyl, and R⁹ is H.

Proviso a) excludes known compounds 6 and 30. Proviso b) excludes known compounds wherein R³ is a group of formula (3a).

The invention also provides novel compounds of the following general Formula (2A), which can also be produced by culturing the novel isolated strains of the invention: 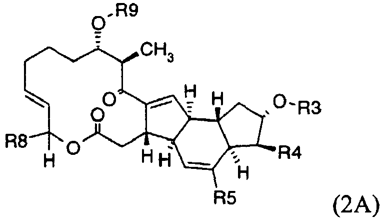 wherein R3, R4, R5, R8, and R9 are as defined for Formula (1A).

Specific novel compounds of Formulas (1A) and (2A) that have been prepared and isolated by culturing the above identified mutant *Saccharopolyspora* sp. strains are identified in Table II.

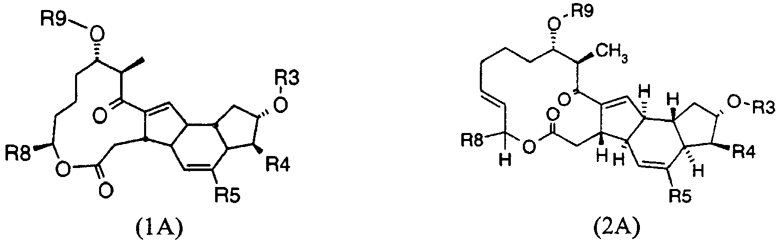

**TABLE II**

| cmpd. no. | Name | formula | R³ * | R⁴ | R⁵ | R⁸ | R⁹ ** |
|---|---|---|---|---|---|---|---|
| 5 | for-(2'-O-desmethyl rham)-I | (1A) | (3b) | H | H | 1-butenyl | (9a) |
| 6 | for-(3'-O-desmethyl rham)-I | (1A) | (3c) | H | H | 1-butenyl | (9a) |
| 32 | for-(4'-*O-*desmethyl-rham)-I | (1A) | (3d) | H | H | 1-butenyl | (9a) |
| 33 | for-(2',3',4'-tri-*O-*desmethyl-rham)-I | (1A) | (3f) | H | H | 1-butenyl | (9a) |
| 34 | for-(4'-*O-*desmethyl-rham)-III | (1A) | (3d) | OH | H | 1-butenyl | (9a) |
| 35 | (4"-*N*-desmethyl-for)-(4'-*O-*desmethyl-rham)-I | (1A) | (3d) | H | H | 1-butenyl | (9c) |
| 36 | for-(4'-*O-*desmethyl-rham)-II | (IA) | (3d) | H | CH₃ | 1-butenyl | (9a) |
| 37 | for-(3',4'-di-*O-*desmethyl-rham)-I | (1A) | (3e) | H | H | 1-butenyl | (9a) |
| 38 | 24-desmethyl-rham-I | (1A) | (3a) | H | H | 1-propenyl | H |
| 39 | (4'-*O*-desmethyl-rham)-I | (1A) | (3d) | H | H | 1-butenyl | H |
| 40 | for-(3',4'-di-*O-*desmethyl-rham)-III | (1A) | (3e) | OH | H | 1-butenyl | (9a) |
| 41 | for-(3'-*O-*desmethyl-rham)-III | (1A) | (3c) | OH | H | 1-butenyl | (9a) |
| 42 | (*N*-oxy-for)-(3',4'-di-*O*-desmethyl-rham)-I | (1A) | (3e) | H | H | 1-butenyl | (9b) |
| 43 | 24-desmethyl-for-(3',4'-di-*O-*desmethyl-rham)-I | (1A) | (3e) | H | H | 1-propenyl | (9a) |
| 44 | for-(3',4'-di-*O-*desmethyl-rham)-II | (1A) | (3e) | H | CH₃ | 1-butenyl | (9a) |
| 137 | (4"-*N*-desmethyl-for)-(3'-*O-*desmethyl-rham)-I | (1A) | (3c) | H | H | 1-butenyl | (9c) |
| 76 | for-(3',4'-di-*O-*desmethyl-rham)-II | (1A) | (3e) | H | CH₃ | 1-butenyl | (9a) |
| 141 | 24-hydroxy-for-(3'-*O*-desmethyl-rham)-I | (1A) | (3c) | H | H | 3-hydroxy-1-butenyl | (9a) |
| 143 | 24,25-dehydro-for-(3'-*O*-desmethyl-rham)-I | (1A) | (3c) | H | H | 1,3-butadienyl | (9a) |
| 148 | 24-desmethyl-for-(3'-*O*-desmethyl-rham)-I | (1A) | (3c) | H | H | 1-propenyl | (9a) |
| 161 | for-(3'-*O-*desmethyl-rham)-IV | (2A) | (3c) | H | H | ethyl | (9a) |
| 163 | for-(3',4'-di-*O-*desmethyl-rham)-IV | (2A) | (3e) | H | H | ethyl | (9a) |
| 165 | 24-keto-22,23-dihydro-for-(3'-*O-*desmethyl-rham)-I | (1A) | (3c) | H | H | butan-3-one | (9a) |
| 166 | 22,23-epoxy-for-(3'-*O*-desmethyl-rham)-I | (1A) | (3c) | H | H | 1,2-epoxy-butyl | (9a) |

| | | | | | | | |
|---|---|---|---|---|---|---|---|
| * formulas (3a)-(3f) are as defined above. ** formulas (9a)-(9i) are as defined above. | | | | | | | |

Two additional compounds with altered bridging of the macrocyclic ring were also isolated. These two compounds had the following structures (10) and (11):

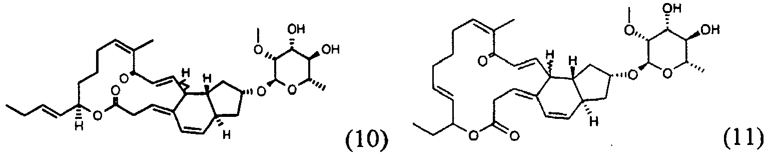

Except for compounds 5 and 6, all of the components listed in Table II are structurally distinct from previously known spinosyns or butenyl-spinosyns. Further, although compounds 5 and 6, in Table I are produced by strain NRRL 30141, they are produced in such small quantities by that strain that isolation is difficult and use of these compounds was up until now very restricted. New strains disclosed in this application produce these compounds in quantities easily isolated by methods disclosed herein.

Strain 30141.2 produces as major products compounds 5 and 33. Strain 30141.3 produces as major products compounds 6, 37, 42, 43, and 44. Strain 30141.4 produces as major products compounds 17, 32, 34, 35, 36, and 39. Strain 30141.5 produces as major products compounds 6, 8, 40, and 41. Strain 30141.8 produces as a major product compound 6, 37, 44, 76, 137, 141, 143, 148, 161,163, 165, and 166. Strain 30141.13 produces as major products compounds 17 and 38.

Strains 30104.2, 30141.3 and 30141.4 between them make as major products derivatives of butenyl-spinosyn (1) which are O-demethylated on the tri-O-methyl-rhamnose moiety at either 2'-position or 3'-position or 4'-position, or in combination such as 3'+4'-positions or 2'+3'+4'-positions. These are exemplified by compounds 5, 6, 32, 37 and 33, respectively. Minor products made by these novel strains include similarly demethylated derivatives in combination with other structural modifications present in metabolites from parent strain 30141, as exemplified by compounds 34 (4'-O-desmethyl, 8-hydroxy), 35 (4'-O-desmethyl, 4"-N-desmethyl), 36 (4'-O-desmethyl, 6-methyl), 43 (3', 4'-di-O-desmethyl, 24-desmethyl) etc. Thus, those skilled in the art would expect these strains to produce all of the metabolites present in the parent strain NRRL 30141 modified with each of the new rhamnose demethylation patterns disclosed herein, in all combinations. Such metabolites can be obtained by performing isolation on extracts from larger batches of broth. Accordingly, the following additional compounds are obtainable from the mutant strains of the present invention:

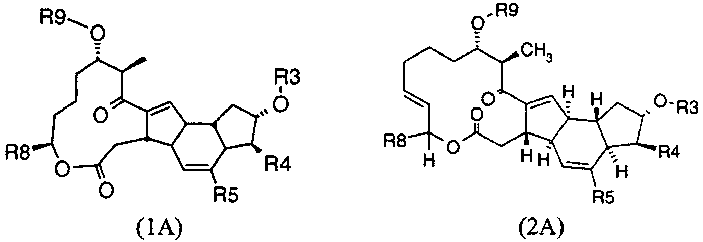

**TABLE III**

| cmpd. no. | formula | R³ * | R⁴ | R⁵ | R⁸ | R⁹ ** |
|---|---|---|---|---|---|---|
| 45 | (1A) | (3d) | H | H | 1-butenyl | (9c) |
| 46 | (1A) | (3d) | H | H | 1-butenyl | (9d) |
| 47 | (1A) | (3d) | H | H | 3-hydroxy-1-butenyl | (9a) |
| 48 | (1A) | (3d) | H | H | 3-hydroxy-1-butenyl | (9c) |
| 49 | (1A) | (3d) | H | H | 1,3-butadienyl | (9a) |
| 50 | (1A) | (3d) | H | H | 1-butenyl | (9e) |
| 51 | (1A) | (3d) | H | H | 1-butenyl | (9f) |
| 52 | (1A) | (3d) | OH | H | 1-butenyl | (9e) |
| 53 | (1A) | (3d) | OH | H | 1-butenyl | (9g) |
| 54 | (1A) | (3d) | H | H | 1-propenyl | (9a) |
| 55 | (1A) | (3d) | H | CH₃ | 1-butenyl | H |
| 56 | (1A) | (3d) | OH | H | 1-butenyl | H |
| 57 | (1A) | (3d) | H | H | 3-hydroxy-1-butenyl | H |
| 58 | (1A) | (3d) | OH | H | 3-hydroxy-1-butenyl | H |
| 59 | (1A) | (3d) | H | H | 1,3-butadienyl | H |
| 60 | (1A) | (3d) | H | H | n-butyl | H |
| 61 | (1A) | (3d) | H | H | 1-butenyl | (9h) |
| 62 | (1A) | (3d) | H | H | 1-butenyl | (9i) |
| 63 | (1A) | (3d) | OH | H | 1,3-butadienyl | (9a) |
| 64 | (1A) | (3d) | OH | CH₃ | 1-butenyl | (9a) |
| 65 | (1A) | (3d) | OH | H | 1-propenyl | (9a) |
| 66 | (1A) | (3e) | H | H | 1-butenyl | (9c) |
| 67 | (1A) | (3e) | H | H | 1-butenyl | (9d) |
| 68 | (1A) | (3e) | H | H | 3-hydroxy-1-butenyl | (9a) |
| 69 | (1A) | (3e) | H | H | 3-hydroxy-1-butenyl | (9c) |
| 70 | (1A) | (3e) | H | H | 1,3-butadienyl | (9a) |
| 71 | (1A) | (3e) | H | H | 1-butenyl | (9e) |
| 72 | (1A) | (3e) | H | H | 1-butenyl | (9f) |
| 73 | (1A) | (3e) | OH | H | 1-butenyl | (9e) |
| 74 | (1A) | (3e) | OH | H | 1-butenyl | (9g) |
| 75 | (1A) | (3e) | H | H | 1-butenyl | H |
| 76 | (1A) | (3e) | H | CH₃ | 1-butenyl | H |
| 77 | (1A) | (3e) | OH | H | 1-butenyl | H |
| 78 | (1A) | (3e) | H | H | 3-hydroxy-1-butenyl | H |
| 79 | (1A) | (3e) | OH | H | 3-hydroxy-1-butenyl | H |
| 80 | (1A) | (3e) | H | H | 1,3-butadienyl | H |
| 81 | (1A) | (3e) | H | H | n-butyl | H |
| 82 | (1A) | (3e) | H | H | 1-butenyl | (9h) |
| 83 | (1A) | (3e) | H | H | 1-butenyl | (9i) |
| 84 | (1A) | (3e) | OH | H | 1,3-butadienyl | (9a) |
| 85 | (1A) | (3e) | OH | CH₃ | 1-butenyl | (9a) |
| 86 | (1A) | (3e) | OH | H | 1-pronenyl | (9a) |
| 87 | (1A) | (3f) | H | H | 1-butenyl | (9b) |
| 88 | (1A) | (3f) | H | H | 1-butenyl | (9c) |
| 89 | (1A) | (3f) | H | H | 1-butenyl | (9d) |
| 90 | (1A) | (3f) | H | CH₃ | 1-butenyl | (9a) |
| 91 | (1A) | (3f) | OH | H | 1-butenyl | (9a) |
| 92 | (1A) | (3f) | H | H | 3-hydroxy-1-butenyl | (9a) |
| 93 | (1A) | (3f) | H | H | 3-hydroxy-1-butenyl | (9c) |
| 94 | (1A) | (3f) | H | H | 1,3-butadienyl | (9a) |
| 95 | (1A) | (3f) | H | H | 1-butenyl | (9e) |
| 96 | (1A) | (3f) | H | H | 1-butenyl | (9f) |
| 97 | (1A) | (3f) | OH | H | 1-butenyl | (9e) |
| 98 | (1A) | (3f) | OH | H | 1-butenyl | (9g) |
| 99 | (1A) | (3f) | H | H | 1-propenyl | (9a) |
| 100 | (1A) | (3f) | H | H | 1-butenyl | H |
| 101 | (1A) | (3f) | H | CH₃ | 1-butenyl | H |
| 102 | (1A) | (3f) | OH | H | 1-butenyl | H |
| 103 | (1A) | (3f) | H | H | 3-hydroxy-1-butenyl | H |
| 104 | (1A) | (3f) | OH | H | 3-hydroxy-1-butenyl | H |
| 105 | (1A) | (3f) | H | H | 1,3-butadienyl | H |
| 106 | (1A) | (3f) | H | H | n-butyl | H |
| 107 | (1A) | (3f) | H | H | 1-butenyl | (9h) |
| 108 | (1A) | (3f) | H | H | 1-butenyl | (9i) |
| 109 | (1A) | (3f) | OH | H | 1,3-butadienyl | (9a) |
| 110 | (1A) | (3f) | OH | CH₃ | 1-butenyl | (9a) |
| 111 | (1A) | (3f) | OH | H | 1-propenyl | (9a) |
| 112 | (1A) | (3b) | H | H | 1-butenyl | (9b) |
| 113 | (1A) | (3b) | H | H | 1-butenyl | (9c) |
| 114 | (1A) | (3b) | H | H | 1-butenyl | (9d) |
| 115 | (1A) | (3b) | H | CH₃ | 1-butenyl | (9a) |
| 116 | (1A) | (3b) | OH | H | 1-butenyl | (9a) |
| 117 | (1A) | (3b) | H | H | 3-hydroxy-1-butenyl | (9a) |
| 118 | (1A) | (3b) | H | H | 3-hydroxy-1-butenyl | (9c) |
| 119 | (1A) | (3b) | H | H | 1,3-butadienyl | (9a) |
| 120 | (1A) | (3b) | H | H | 1-butenyl | (9e) |
| 121 | (1A) | (3b) | H | H | 1-butenyl | (9f) |
| 122 | (1A) | (3b) | OH | H | 1-butenyl | (9e) |
| 123 | (1A) | (3b) | OH | H | 1-butenyl | (9g) |
| 124 | (1A) | (3b) | H | H | 1-propenyl | (9a) |
| 125 | (1A) | (3b) | H | CH₃ | 1-butenyl | H |
| 126 | (1A) | (3b) | OH | H | 1-butenyl | H |
| 127 | (1A) | (3b) | H | H | 3-hydroxy-1-butenyl | H |
| 128 | (1A) | (3b) | OH | H | 3-hydroxy-1-butenyl | H |
| 129 | (1A) | (3b) | H | H | 1,3-butadienvl | H |
| 130 | (1A) | (3b) | H | H | n-butyl | H |
| 131 | (1A) | (3b) | H | H | 1-butenyl | (9h) |
| 132 | (1A) | (3b) | H | H | 1-butenyl | (9i) |
| 133 | (1A) | (3b) | OH | H | 1,3-butadienyl | (9a) |
| 134 | (1A) | (3b) | OH | CH₃ | 1-butenyl | (9a) |
| 135 | (1A) | (3b) | OH | H | 1-propenyl | (9a) |
| 136 | (1A) | (3c) | H | H | 1-butenyl | (9b) |
| 137 | (1A) | (3c) | H | H | 1-butenyl | (9c) |
| 138 | (1A) | (3c) | H | H | 1-butenyl | (9d) |

| cmpd. no. | formula | R³* | R⁴ | R⁵ | R⁸ | R⁹ ** |
|---|---|---|---|---|---|---|
| 139 | (1A) | (3c) | H | CH₃ | 1-butenyl | (9a) |
| 140 | (1A) | (3c) | OH | H | 1-butenyl | (9a) |
| 141 | (1A) | (3c) | H | H | 3-hydroxy-1-butenyl | (9a) |
| 142 | (1A) | (3c) | H | H | 3-hydroxy-1-butenyl | (9c) |
| 143 | (1A) | (3c) | H | H | 1,3-butadienyl | (9a) |
| 144 | (1A) | (3c) | H | H | 1-butenyl | (9e) |
| 145 | (1A) | (3c) | H | H | 1-butenyl | (9f) |
| 146 | (1A) | (3c) | OH | H | 1-butenyl | (9e) |
| 147 | (1A) | (3c) | OH | H | 1-butenyl | (9g) |
| 148 | (1A) | (3c) | H | H | 1-propenyl | (9a) |
| 149 | (1A) | (3c) | H | CH₃ | 1-butenyl | H |
| 150 | (1A) | (3c) | OH | H | 1-butenyl | H |
| 151 | (1A) | (3c) | H | H | 3-hydroxy-butenyl | H |
| 152 | (1A) | (3c) | OH | H | 3-hydroxy-1-butenyl | H |
| 153 | (1A) | (3c) | H | H | 1,3-butadienyl | H |
| 154 | (1A) | (3c) | H | H | n-butyl | H |
| 155 | (1A) | (3c) | H | H | 1-butenyl | (9h) |
| 156 | (1A) | (3c) | H | H | 1-butenyl | (9i) |
| 157 | (1A) | (3c) | OH | H | 1,3-butadienyl | (9a) |
| 158 | (1A) | (3c) | OH | CH₃ | 1-butenyl | (9a) |
| 159 | (1A) | (3c) | OH | H | 1-propenyl | (9a) |
| 167 | (1A) | (3d) | H | H | butan-3-one | (9a) |
| 168 | (1A) | (3d) | H | H | 1,2-epoxy-butyl | (9a) |
| 169 | (1A) | (3e) | H | H | butan-3-one | (9a) |
| 170 | (1A) | (3e) | H | H | 1,2-epoxy-butyl | (9a) |
| 171 | (1A) | (3e) | H | H | butan-3-one | (9a) |
| 172 | (1A) | (3e) | H | H | 1,2-epoxy-butyl | (9a) |
| 173 | (1A) | (3b) | H | H | butan-3-one | (9a) |
| 174 | (1A) | (3b) | H | H | 1,2-epoxy-butyl | (9a) |

| | | | | | | |
|---|---|---|---|---|---|---|
| * formulas (3a)-(3f) are as defined above. ** formulas (9a)-(9i) are as defined above. | | | | | | |

Scale-up of strain 30141.8 allowed the isolation of several new derivatives of butenyl-spinosyn (1) with novel substitution at C-21 as exemplified by compounds 165 (24-keto-22-23-dihydro-for-(3'-O-desmethyl-rham)-I) and 166 (22,23-epoxy-for-(3'-O-desmethyl-rham)-I). Thus, those skilled in the art would expect the parent strain to produce all of the metabolites present in the parent strain NRRL 30141 modified with each of the new C-21 modification patterns disclosed herein, in all combinations (Table IIIb). Such metabolites can be obtained by performing isolation on extracts from larger batches of broth.

**TABLE IIIb**

| cmpd. no. | formula† | R³ * | R⁴ | R⁵ | R⁸ | R⁹ ** |
|---|---|---|---|---|---|---|
| 175 | (1A) | (3a) | H | H | butan-3-one | (9a) |
| 176 | (1A) | (3a) | H | H | 1,2-epoxy-butyl | (9a) |
| 177 | (1A) | (3a) | OH | H | butan-3-one | (9a) |
| 178 | (1A) | (3a) | OH | H | 1,2-epoxy-butyl | (9a) |
| 179 | (1A) | (3a) | H | CH₃ | butan-3-one | (9a) |
| 180 | (1A) | (3a) | H | CH₃ | 1,2-epoxy-butyl | (9a) |
| 181 | (1A) | (3a) | OH | CH₃ | butan-3-one | (9a) |
| 182 | (1A) | (3a) | OH | CH₃ | 1,2-epoxy-butyl | (9a) |
| 183 | (1A) | (3a) | H | H | butan-3-one | (9b) |
| 184 | (1A) | (3a) | H | H | butan-3-one | (9c) |
| 185 | (1A) | (3a) | H | H | butan-3-one | (9d) |
| 186 | (1A) | (3a) | H | H | butan-3-one | (9e) |
| 187 | (1A) | (3a) | H | H | butan-3-one | (9f) |
| 188 | (1A) | (3a) | OH | H | butan-3-one | (9e) |
| 189 | (1A) | (3a) | OH | H | butan-3-one | (9g) |
| 190 | (1A) | (3a) | H | CH₃ | butan-3-one | H |
| 191 | (1A) | (3a) | OH | H | butan-3-one | H |
| 192 | (1A) | (3a) | H | H | butan-3-one | (9h) |
| 193 | (1A) | (3a) | H | H | butan-3-one | (9i) |
| 194 | (1A) | (3a) | H | H | 1,2-epoxy-butyl | (9b) |
| 195 | (1A) | (3 a) | H | H | 1,2-epoxy-butyl | (9c) |
| 196 | (1A) | (3a) | H | H | 1,2-epoxy-butyl | (9d) |
| 197 | (1A) | (3a) | H | H | 1,2-epoxy-butyl | (9e) |
| 199 | (1A) | (3a) | H | H | 1,2-epoxy-butyl | (9f) |
| 200 | (1A) | (3a) | OH | H | 1,2-epoxy-butyl | (9e) |
| 201 | (1A) | (3a) | OH | H | 1,2-epoxy-butyl | (9g) |
| 202 | (1A) | (3a) | H | CH₃ | 1,2-epoxy-butyl | H |
| 203 | (1A) | (3a) | OH | H | 1,2-epoxy-butyl | H |
| 204 | (1A) | (3a) | H | H | 1,2-epoxy-butyl | (9h) |
| 205 | (1A) | (3a) | H | H | 1,2-epoxy-butyl | (9i) |
| 206 | (1A) | H | H | H | butan-3-one | (9a) |
| 207 | (1A) | H | H | H | 1,2-epoxy-butyl | (9a) |
| | | | | | | |
| | | | | | | |

| | | | | | | |
|---|---|---|---|---|---|---|
| t formula 1A is as defined above * formulas (3a)-(3f) are as defined above. * formulas (9a)-(9i) are as defined above. | | | | | | |

Another aspect of this invention is a process for producing compounds of Formula (1A), which comprises culturing a strain of *Saccharopolyspora sp.* selected from strains 30141.2, 30141.3, 30141.4, 30141.5, 30141.8, and 30141.13 in a suitable medium to produce the desired compounds. The Formula (1A) compounds are extracted from the fermentation broth and from the mycelium with polar organic solvents. The compounds may be further purified by techniques well-known in the art, such as column chromatography.

Because strains 30141.2, 30141.3, 30141.4, 30141.5, 30141.8, and 30141.13 are newly discovered strains, this invention further provides for a biologically purified culture of these microorganisms. The novel strains were deposited in accordance with the terms of the Budapest treaty at the Midwest Area Regional Research Center, Agricultural Research Service, United States Department of Agriculture, 815 North University Street, Peoria, IL 61604, on the dates indicated in the following table:

| strain | deposit number | deposit date |
|---|---|---|
| 30141.2 | NRRL 30424 | March 8, 2001 |
| 30141.3 | NRRL 30423 | March 8, 2001 |
| 30141.4 | NRRL 30422 | March 8, 2001 |
| 30141.5 | NRRL 30438 | March 15, 2001 |
| 30141.8 | NRRL 30421 | March 8, 2001 |
| 30141.13 | NRRL 30437 | March 15, 2001 |

Formula 1A compounds where R⁹ is other than H, hereinafter referred to as Formula 1B compounds, are useful for the control of mites and insects, particularly *Lepidoptera, Coleoptera, Homoptera* and *Diptera* species. Therefore, insecticidal and miticidal compositions and methods for reducing the populations of insects and mites using these compounds are also a part of this invention.

Compounds of Formula 1A are also useful as intermediates in the preparation of insecticidal and miticidal compounds. For example, these compounds can be alkylated or acylated at any free rhamnose hydroxyl group. The alkylation or acylation may be carried out by chemical synthesis or by microbial bioconversion, using procedures described, for example, in WO 97/00265.

### DETAILED DESCRIPTION OF INVENTION

### CULTURE DESCRIPTION

The newly isolated strains derived from *Saccharopolyspora* sp. LW 107129 (NRRL30141) were grown on seven agar plating media and compared for growth, reverse color, aerial hyphae production, spore mass color and pigment production. In addition, the mutants were tested for the ability to ferment 14 sugars. In both morphological and physiological testing, no significant differences were observed between NRRL 30141 as disclosed in WO 01/19840 and any of the cultures disclosed herein (30141.2, 30141.3, 30141.4, 30141.5, 30141.8, or 30141.13).

One aspect of the present invention is the preparation of a compound of Formula 1A by culturing a 3'-ODM-rham-producing strain such as 30141.3, 30141.5, 30141.8 or a 3'-ODM-rham-producing mutant thereof. A "3'-ODM-rham-producing mutant" is a strain derived from any one of the butenyl-spinosyn producing strains of NRRL 30141 or ODM-rham-producing strains of NRRL 30141, which is capable of producing recoverable amounts of 3'-O-desmethyl-rham compounds.

Another aspect of the present invention is the preparation of a compound of Formula 1A by culturing a 2'-ODM-rham-producing strain such as 30141.2 or a 2'-ODM-rham-producing mutant thereof. A "2'-ODM-rham-producing mutant" is a strain derived from any one of the butenyl-spinosyn producing strains of NRRL 30141 or of the ODM-rham-producing strains ofNRRL 30141, which is capable of producing recoverable amounts of 2'-O-desmethyl-rham compounds.

A still further aspect of the present invention is the preparation of a compound of Formula (1A) produced by culturing a 4'-ODM-rham-producing strain such as 30141.4 or a 4'-ODM-rham-producing mutant thereof. A "4'-ODM-rham-producing mutant" is a strain derived from any one of the butenyl-spinosyn producing strains of NRRL 30141 or of the 4'-ODM-rham-producing strains of NRRL 30141, which is capable of producing recoverable amounts of 4'-O-desmethyl-rham compounds.

Formula 1A compounds may be separated from the culture medium in which they are produced using various isolation and purification procedures, which are well understood in the art. For economy in production, optimal yield, and ease of product isolation, certain culture media are preferred. For example the preferred carbon source in large-scale fermentation is glucose, although ribose, fructose, glycerol, mannose, mannitol, ribose, soluble starch, potato dextrin, oils such as soy oil and the like can also be used. Preferred nitrogen sources are cottonseed flour, soybean flour, peptonized casein and corn steep liquor, although soy peptone, yeast extract, enzyme hydrolized casein, beef extract, and the like can be used. Among the nutrient inorganic salts which can be incorporated in the culture media are the customary soluble salts capable of yielding zinc, sodium, potassium, magnesium, calcium, ammonium, chloride carbonate, sulfate, nitrate, phosphate and like ions. Essential trace elements commonly occur as impurities in other substituents of the medium in amounts sufficient to meet the growth requirements of the organism, although salts capable of yielding molybdenum, cobalt, copper, manganese, boron, iron and the like may be added.

Usually if foaming is a problem, small amounts of an antifoam agent such as polypropylene glycol or other antifoams commonly used by those skilled in the art may be added to large-scale fermentation media. Additionally, oil may be added if foaming develops.

For production of substantial quantities of Formula 1A compounds, submerged aerobic fermentation is preferred, however, small quantities of a Formula 1A compound may be obtained by shake flask culture. It is preferable to use a vegetative inoculum for the inoculation of large bioreactors. The vegetative inoculum is prepared by inoculating a small volume of culture medium from a stock culture preserved in liquid nitrogen or an ultra-low temperature freezer to obtain a fresh, actively growing culture of that organism. The vegetative culture is then transferred to a larger bioreactor. The vegetative inoculum medium can be the same as that used for fermentation, but other media are also suitable.

The Formula 1A compound is produced by the ODM-rham-producing strains when grown at temperatures between about 24° C and about 33° C. Optimum temperatures for production appear to be about 28° C to about 31° C. As is customary in submerged aerobic culture processes, sterile air is blown into the vessel from the bottom, while the medium is stirred with turbine impellers. In general aeration rate should be sufficient to maintain the level of dissolved oxygen required by the organism.

Production of Formula 1A compounds can be followed during the fermentation by testing extracts of the broth. A preferred method for following the production is analysis of the broth extracts by high performance liquid chromatography (HPLC). A more preferred method for following the production is analysis of the broth extracts by HPLC-mass spectrometry (LC-MS). Suitable systems for analysis are described in Examples 1 and 2.

Following the production in shake flasks or in stirred reactors, the Formula 1A compounds can be recovered from the fermentation medium by methods known to those skilled in the art. The compounds created during fermentation of the butenyl-spinosyn producing strain occur in both the mycelia and the broth. In the absence of oil in the fermentation, more efficient recovery of the Formula 1A compound is accomplished by initially filtering the whole broth to separate the mycelium from the broth. The Formula (1A) compounds are lipophilic; therefore, when oil is used in the fermentation, a substantial amount of the Formula 1A compounds may be in the broth.

A preferred technique for isolating the Formula (1A) compounds from the broth involves adding an equal volume of ethanol to the whole broth, shaking for 1-2 hours to complete extraction and centrifuging to separate the extracted cell mass. The Formula (1A) compounds are then extracted by partitioning the aqueous ethanolic broth extract with dichloromethane, which is dried under vacuum. The Formula (1A) compounds are purified from the dried dichloromethane phase by preparative HPLC, as described in more detail in Example 4.

A more preferred technique for isolating Formula (1A) compounds (where R⁹ is a basic nitrogen-containing moiety) from the broth involves adding methyl isobutyl ketone (MIBK) to the broth. After shaking for 1-2 hours, the broth is centrifuged and the MIBK extract is separated. The MIBK extract is partitioned using acidic aqueous buffer at pH 3, the aqueous phase is adjusted to pH 10 using ammonium hydroxide then extracted using diethyl ether (DEE). The DEE phase is dried under vacuum and the Formula (1A) compounds are separated and purified by preparative HPLC, as described in more detail in Example 5.

Alternatively, the culture solids, including medium constituents and mycelium, can be used without extraction or separation, but preferably after removal of water, as a source of the Formula (1A) compounds. For example, after production of Formula (1A) compounds, the whole fermentation broth can be dried by lyophilization, by drum-drying, or by azeotropic distillation and drying. The dried broth can then be used directly, for example by mixing it directly into feed pre-mix or into formulations for sprays and powders.

### EXAMPLE 1

### LC-UV Method for Analysis of Fermentation Broth for Butenyl-Spinosyn Metabolites Containing O-Demethyl-Rhamnose

The following HPLC methods are useful for monitoring a fermentation for the production of compounds of Formula (1A) and other components:

Add a volume of denatured ethanol equal to that of fermentation broth. Shake the mixture for 1 hour, then centrifuge to remove the bulk cell debris. Microfuge a 1-mL aliquot, then analyze the clarified extract by one of the following HPLC systems:

HPLC system 1: Column stationary phase: 250 x 4.6-mm column, base-deactivated silica gel, 5 µm C8 (Hypersil™-C8-BDS). Mobile phase: 10 mM ammonium acetate-methanol-acetonitrile linear gradient summarized below:

**TABLE IVa**

| Time (mins) | Percent solvent A | Percent solvent B |
|---|---|---|
| 0 | 100 | 0 |
| 20 | 0 | 100 |
| 25 | 0 | 100 |
| 30 | 100 | 0 |
| 35 | 100 | 0 |

where solvent A is 10 mM ammonium acetate and solvent B is methanol-acetonitrile (1:1).
Flow rate: 1 mL/min
Detection: UV at 244 nm
Retention times as summarized in Table V:

**TABLE Va**

| Compound | Retention time (min) |
|---|---|
| (5) | 23.5 |
| (6) | 23.6 |
| (32) | 23.5 |
| (33) | 22.4 |
| (34) | 21.1 |
| (35) | 22.6 |
| (36) | 23.8 |
| (37) | 23.1 |
| (38) | 21.6 |
| (39) | 21.4 |
| (40) | 20.4 |
| (41) | 21.3 |
| (42) | 22.4 |
| (43) | 22.4 |
| (44) | 23.6 |

HPLC system 2: Column stationary phase: 250 x 4.6-mm column, base-deactivated silica gel, 5 µm C8 (Hypersil™-C8-BDS). Mobile phase: 10 mM ammonium acetate - acetonitrile linear gradient summarized below:

**TABLE IVb**

| Time (mins) | Percent solvent A | Percent solvent B |
|---|---|---|
| 0 | 100 | 0 |
| 20 | 0 | 100 |
| 25 | 0 | 100 |
| 30 | 100 | 0 |
| 35 | 100 | 0 |

where solvent A is 10 mM ammonium acetate and solvent B is acetonitrile.
Flow rate: 1 mL/min
Detection: UV at 244 nm

Retention times as summarized in Table Vb:

**TABLE Vb**

| Compound | Retention time (min) |
|---|---|
| (165) | 17.3 |
| (166) | 18.5 |
| (10) | 18.7 |
| (11) | 18.9 |

HPLC system 3: Column stationary phase: 250 x 4.6-mm column, basedeactivated silica gel, 5 µm C18 (Hypersil™-C18-BDS). Mobile phase: 10 mM ammonium acetate - acetonitrile linear gradient summarized below:

**TABLE IVc**

| Time (mins) | Percent solvent A | Percent solvent B |
|---|---|---|
| 0 | 100 | 0 |
| 20 | 0 | 100 |
| 25 | 0 | 100 |
| 30 | 100 | 0 |
| 35 | 100 | 0 |

where solvent A is 10 mM ammonium acetate and solvent B is acetonitrile.
Flow rate: 1 mL/min
Detection: UV at 244 nm

Retention times as summarized in Table Vc:

**TABLE Vc**

| Compound | Retention time (min) |
|---|---|
| (76) | 22.7 |
| (163) | 23.4 |

### EXAMPLE 2

### LC-MS Method for Analysis of Fermentation Broth for Butenyl-Spinosyn Metabolites Containing O-Demethyl-Rhamnose

The LC method described in Example 1, except with the addition of an electrospray (ESI) mass spectrometer attached to analyze the HPLC eluent, is useful for monitoring a fermentation for the production of compounds of Formula (1A) and other components. Such a system was also used for determining molecular weights of the purified factors, by deduction from the electrospray adduct ions. These data are summarized in Table VI.

Add a volume of denatured ethanol equal to that of fermentation broth. Shake the mixture for 1 hour, then centrifuge to remove the bulk cell debris. Microfuge a 1-mL aliquot, then analyze the clarified extract by the following LC-MS system:

HPLC system: Column stationary phase: 250 x 4.6-mm column, base-deactiviated silica gel, 5 µm C8 (Hypersil™-C8-BDS). Mobile phase: 10 mM ammonium acetate-methanol-acetonitrile linear gradient summarized below:

**TABLE VI**

| Time (mins) | Percent solvent A | Percent solvent B |
|---|---|---|
| 0 | 100 | 0 |
| 20 | 0 | 100 |
| 25 | 0 | 100 |
| 30 | 100 | 0 |
| 35 | 100 | 0 |

where solvent A is 10 mM ammonium acetate and solvent B is methanol-acetonitrile (1:1).
Flow rate: 1 mL/min; split after UV detector such that MS:waste ratio is approx. 5:95.
Detection: positive ESI acquired in low and high cone voltage modes

Characteristic mass spectrometry ions as summarized in Table VII.

**TABLE VII**

| Compound | m/z for [M+H]^{+a} | m/z for secondary ion^{b} |
|---|---|---|
| 5 | 744.3 | 142.0 |
| 6 | 744.6 | 142.1 |
| 32 | 744.5 | 142.1 |
| 33 | 716.4 | 142.1 |
| 34 | 760.3 | 142.0 |
| 35 | 730.5 | 128.1 |
| 36 | 758.6 | 142.1 |
| 37 | 730.2 | 142.0 |
| 38 | 603.4 | [M+acetate]⁺ = 661.5 |
| 39 | 603.4 | [M+acetate]⁺ = 661.4 |
| 40 | 746.4 | 142.1 |
| 41 | 760.4 | 142.1 |
| 42 | 746.2 | 142.1 |
| 43 | 716.3 | 142.0 |
| 44 | 744.4 | 142.1 |
| 137 | 730.5 | 128.0 |
| 141 | 760.5 | 142.0 |
| 143 | 742.5 | 142.0 |
| 148 | 730.4 | 142.0 |
| 161 | 744.4 | 142.0 |
| 163 | 729.9 | NO^{c} |
| 165 | 760.5 | NO^{c} |
| 166 | 760.5 | NO^{c} |
| Compound of formula (10) | 571.5 | NO^{c} |
| Compound of formula (11) | 571.5 | NO^{c} |

| | | |
|---|---|---|
| ^{a} m/z for parent ion observed under +ESI mode, low cone voltage ^{b} m/z for most abundant fragment or adduct ion observed under +ESI, high cone voltage mode ^{c} NO = not observed under experimental conditions used. | | |

### EXAMPLE 3

### Preparation Of Butenyl-Spinosyn Metabolites Containing O-Demethyl-Rhamnose Through Fermentation

Metabolites of Formula (1A) containing O-demethyl-rhamnose are produced by cultivation of the desired strain of *Saccharopolyspora* chosen from one of the following strains 30141.2, 30141.3, 30141.4, 30141.5, or 30141.8, in fermentation medium as described below. A 1.8-mL frozen vegetative culture was thawed, inoculated into 100 mL vegetative media in a 500-mL Erlenmeyer flask and grown at 30° C shaking at 150 rpm for 48-72 hours.

**TABLE VIII**

| Vegetative Medium (per liter of water) | |
|---|---|
| Ingredient | Amount (g) |
| Dextrose | 9.0 |
| Trypticase Soy Broth | 30.0 |
| Yeast Extract | 3.0 |
| Magnesium Sulfate. 7 H₂O | 2.0 |

### Shake Flask Fermentation

Fifty milliliters of mature first stage seed was used to inoculate 800-mL fermentation medium in a 2 liter TunAir™ fermentation flask. Production of Formula (1A) compounds can be monitored in shake flask fermentation by the HPLC methods disclosed in Examples 1 and 2.

**TABLE IX**

| Fermentation Medium (per liter of water) | |
|---|---|
| Ingredient | Amount (g) |
| Dextrose | 80.0 |
| Cottonseed Flour | 32.0 |
| Soybean Flour | 8.0 |
| Corn Steep Powder | 8.0 |
| Calcium Carbonate | 5.0 |
| Yeast Extract | 2.0 |

Fermentation was maintained at 30° C, 200 rpm (50 mm stroke) for 7-12 days.

### B. Stirred Reactor Fermentation

Sixty milliliters of mature first stage vegetative culture was used to inoculate a secondary vegetative culture of 1 liter vegetative medium in a 2-liter culture flask. This culture was incubated at 30° C for 48 hours shaking at 195 rpm. The mature second stage seed was used to inoculate 70 liters of fermentation medium in a stirred tank fermentation vessel. Fermentation was maintained at 30° C, 400 rpm for 7-14 days. Production of Formula (1A) compounds can be monitored in stirred tank fermentation by the HPLC methods disclosed in Examples 1 and 2.

Mature fermentation beer can be extracted with a suitable solvent and the metabolites recovered by salt formation and/or chromatographic separation, as disclosed in Examples 4 and 5.

### EXAMPLE 4

### Isolation of Insecticidally-Active Metabolites from Culture Broth using Neutral Method

The following example shows how metabolites were isolated when a mixture of Formula (1A) compounds (where R⁹ = H and/or basic-nitrogen-containing sugar) were purified. The example gives a specific procedure used to isolate compounds 39 and 17, but this would be applicable to any such mixture when practiced by one skilled in the art.

The total culture of strain 30141.4, i.e. cells plus broth, from fermentation of 800 mL of inoculated medium had a total volume of approximately 600 mL after fermentation was complete. The sample was extracted with an equal volume of denatured ethanol by shaking vigorously then allowing to stand at room temperature for two hours. The cell debris was removed by centrifugation and the 1.2 liters of 50% aqueous ethanol extract was partitioned using dichloromethane (DCM; 2 x 600 mL). The DCM extract was concentrated under vacuum to give 470 mg of residue. The residue was dissolved in methanol and chromatographed by repeated injections on preparative HPLC under the following conditions:

HPLC system: Column stationary phase: 250 x 10-mm column, base-deactiviated silica gel, 5 µm C18 (Hypersil™-C18-BDS). Mobile phase: 10 mM ammonium acetate-methanol-acetonitrile (25:37.5:37.5). Flow conditions: isocratic elution at 5 mL/min. Detection: UV at 244 nm.

Half of the dried DCM extract was separated preparatively using this method, collecting the regions eluting at approximately 9 minutes and 13.5 minutes. These were dried, to give pure compound 39 (1.7 mg) and compound 17 (0.8 mg), respectively.

### EXAMPLE 5

### Isolation of Insecticidally-Active Metabolites from Culture Broth using Acid Back-Extraction Method

The following example shows how metabolites were isolated when the compounds of interest were a mixture of Formula (1A) compounds (where R⁹ = basic-nitrogen-containing sugar). The example gives a specific procedure used to isolate compounds 6, 37, 42, 43 and 44, but this would be applicable to any such mixture when practiced by one skilled in the art.

The total culture of strain 30141.3, i.e. cells plus broth, from fermentation of six liters of inoculated medium had a total volume of approximately five liters after fermentation was complete. The sample was extracted with methyl isobutyl ketone (MIBK; 2 x 2.5 liters) by shaking continuously for one hour. The cell debris was removed by centrifugation. The MIBK extract was partitioned using aqueous ammonium acetate/formic acid buffer (10 mM; pH 3; 2 x 1 liter). The pH of the aqueous extract was adjusted to 10 using 30% ammonium hydroxide solution and the solution was extracted with diethyl ether (DEE; 2 x 1 liter). The DEE was passed through a paper filter then dried under vacuum, giving 127 mg of residue, referred to as DEE-1. This residue was dissolved in methanol (500 µL) and chromatographed by repeated injections on preparative HPLC under the following conditions:

HPLC system: Column stationary phase: 250 x 10-mm column, base-deactiviated silica gel, 5-µm C18 (Hypersil™-C18-BDS). Mobile phase: 10 mM ammonium acetate-methanol-acetonitrile (20:40:40). Flow conditions: isocratic elution at 5 mL/min.
Detection: UV at 244 nm.

The DEE-1 extract was separated preparatively using this method, collecting the regions eluting at approximately 10.2 minutes, 10.8 minutes, 13.5 minutes and 17.5 minutes. These were dried, to give pure compounds 42 (0.2 mg), 43 (1.9 mg), 37 (23.4 mg) and a mixture of compounds 6 and 44 (3.1 mg), respectively.

The MIBK phase remaining from the initial acidic buffer extraction described above was concentrated to dryness under vacuum. The residue was dissolved in DEE (200 mL) and partitioned against water (2 x 100 mL; adjusted to pH 3 using 1N-HCl). The aqueous phase was adjusted to pH 10 using 30% ammonium hydroxide solution and the solution was extracted with DEE (2 x 100 mL). The DEE was passed through a paper filter then dried under vacuum, giving 106 mg of residue, referred to as DEE-2. This residue was dissolved in methanol (500 µL) and chromatographed by repeated injections on preparative HPLC under the following conditions:

HPLC system: Column stationary phase: 250 x 10-mm column, base-deactiviated silica gel, 5-µm C18 (Hypersil™-C18-BDS). Mobile phase: 10 mM ammonium acetate-methanol-acetonitrile (20:40:40). Flow conditions: isocratic elution at 5 mL/min. Detection: UV at 244 nm.

The DEE-2 extract was separated preparatively using this method, collecting the regions eluting at approximately 13.5 minutes and 17.5 minutes. These were dried, to give pure compound 37 (22.6mg) and a mixture of compounds 6 and 44 (10.6 mg), respectively.

The compound mixtures obtained from the first step chromatographic separations of DEE-1 and DEE-2 were pooled and dried. The dried sample was dissolved in methanol (500 µL) and chromatographed by repeated injections on preparative HPLC under the following conditions:
HPLC system: Column stationary phase: 250 x 10-mm column, base-deactiviated silica gel, 5-µm C18 (Hypersil™-C18-BDS). Mobile phase: 10 mM ammonium acetate-acetonitrile (30:70). Flow conditions: isocratic elution at 5 mL/min. Detection: UV at 244 nm.

The compound mixture was separated preparatively using this method, collecting the regions eluting at approximately 12 minutes and 16 minutes. These were dried, to give pure compound 44 (4.0 mg) and pure compound 6 (8.4 mg), respectively.

### Spectroscopic Characteristics

The chemical structures of the new components were determined by spectroscopic methods, including nuclear magnetic resonance spectroscopy (NMR), mass spectrometry (MS, as summarized in Table VII), ultraviolet spectroscopy (UV) and by comparison to for-rham-I (1) and related analogs. The NMR spectra of all compounds were acquired using common NMR techniques, and compared to the spectra derived from other related structures. Any differences between the compounds and the NMR spectra of (for example) compound 1 were investigated using standard inverse 2D NMR experiments such as COSY, HSQC, and HMBC. For the purposes of identifying the derivatives of the C-9 sugar substituent, the numbering system used is as follows.

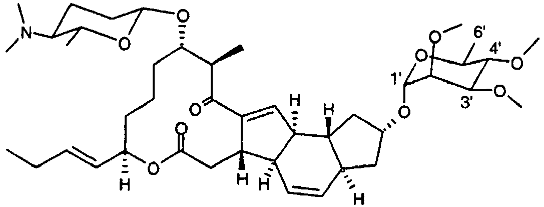

Distinguishing NMR spectroscopic features used to identify the Formula (1A) compounds were as follows: The compounds were readily identified by comparing the 1H spectrum with that for compound 1. Site of demethylation could be easily determined by comparison, and confirmed by appropriate use of 2D NMR technology. Any other features (for example compounds 36 and 40) were also readily identified by comparison with previously claimed molecules (7 and 8). The relevant changes in the NMR spectra were exemplified by comparing the proton chemical shift for the protons of the C-9 sugar between compounds 5, 6, 32, 33, and 37, and those for compound 1. Downfield shifts were noted in all cases where the methyl was not present.

**TABLE X**

| | Proton Chemical Shift (ppm) | | | |
|---|---|---|---|---|
| Factor | 2' | 3' | 4' | 5' |
| (1) For-rham-I | 3.50 | 3.46 | 3.11 | 3.55 |
| (5) For-(2'-*O*-desmethyl-rham)-I | **3.96** | 3.45 | 3.08 | 3.62 |
| (6) For-(3'-*O*-desmethyl-rham)-I | 3.42 | **3.83** | 2.98 | 3.56 |
| (32) For-(4'-*O*-desmethyl-rham)-I | 3.58 | 3.42 | **3.59** | 3.50 |
| (37) For-(3',4'-di-*O*-desmethyl-rham)-I | 3.42 | **3.72** | **3.38** | 3.53 |
| (33) For-(2',3',4'-tri-*O*-desmethyl-rham)-I | **3.89** | **3.78** | **3.46** | 3.58 |

### Insecticide and Miticide Activity

Compounds of Formula (1B) (i.e. compounds of Formula (1A) compounds where R⁹ is a sugar moiety) are useful for the control of insects and mites. Therefore, a further aspect of the present invention is directed to methods for inhibiting an insect or mite which comprises applying to the locus of the insect or mite an insect- or mite-inhibiting amount of a Formula (1B) compound.

The "locus" of the insect or mite refers to the environment in which the insect or mite lives or where its eggs are present, including the air surrounding it, the food it eats, or objects which it contacts. For example, plant-ingesting insects or mites can be controlled by applying the active compound to plant parts which the insects or mites eat or inhabit, particularly the foliage.

By the term "inhibiting an insect or mite" it is meant that there is a decrease in the number of living insects or mites or a decrease in the number of eggs. The extent of reduction accomplished by a compound depends, of course, on the application rate of the compound, the particular compound used, and the target insect or mite species. At least an insect-inactivating or mite-inactivating amount should be used. By "inactivating amount" it is meant that an amount of compound is used to cause measurable reduction in the treated insect or mite population. Typically from about 1 to about 1,000 ppm (or 0.01 to 1 Kg/acre) of compound is used.

The compounds show activity against a number of insects and mites. More specifically, the compounds show activity against members of the insect order Lepidoptera such as the beet armyworm, tobacco budworm, codling moth and cabbage looper. Other typical members of this order include the southern armyworm, cutworms, clothes moths, Indian meal moth, leaf rollers, corn earworm, cotton bollworm, European corn borer, imported cabbage worm, pink bollworm, bagworms, Eastern tent caterpillar, sod webworm, and fall armyworm.

The compounds also show activity against members of the order *Coleoptera* (the beetles and weevils such as the Colorado potato beetle, spotted and striped cucumber beetles, Japanese beetle, and boll weevil) and *Diptera* (the true flies such as the house fly, mosquitoes, fruit flies, stable and horn flies, and leaf miners)

The compounds also show activity against members of the order *Hempitera* (true bugs such as plant bugs, stink bugs, and chinch bugs), *Homoptera* (such as the aphids, leafhoppers, planthoppers, whiteflies, scales, and mealybugs), *Thysanoptera* (thrips), *Orthoptera* (such as cockroaches, grasshoppers, and crickets), *Siphonaptera* (fleas), *Isoptera* (termites), and members of the *Hymenoptera* order *Formicidae* (ants).

The compounds also show activity against the two-spotted spider mite, which is a member of the Arachnid order *Acarina.* Other typical members of this order include plant parasites such as the citrus red mite, European red mite, and citrus flat mite, and animal parasites such as the mange mite, scab mite, sheep scab mite, chicken mite, scalyleg mite, depluming mite and dog follicle mite.

Specific representative anthropod pests which can be controlled by the present compounds include the following: *Amblyomma americanum* (Lone-star tick), *Amblyomma maculatum* (Gulf Coast tick), *Argas persicus* (fowl tick), *Boophilus microplus* (cattle tick), *Chorioptes* spp. (mange mite), *Demodex bovis* (cattle follicle mite), *Demodex canis* (dog follicle mite), *Dermacentor andersoni* (Rocky Mountain spotted fever tick), *Dermacentor variabilis* (American dog tick), *Dermanyssus gallinae* (chicken mite), *Ixodes ricinus* (common sheep tick), *Knemidokoptes gallinae* (deplumming mite), *Knemidokoptes mutans* (scaly-leg mite), *Otobius megnini* (ear tick), *Psoroptes equi* (scab mite), *Psoroptes ovis* (scab mite), *Rhipicephalus sanguineus* (brown dog tick), *Sarcoptes scabiei* (mange mite), *Aedes* (mosquitoes), *Anopheles* (mosquitoes), *Culex* (mosquitoes), *Culiseta, Bovicola bovis* (cattle biting louse), *Callitroga homnivorax* (blowfly), *Chrysops* spp. (deer fly), *Cimex lectularius* (bed bug), *Cochliomyia* spp. (screwworm), *Ctenocephalides canis* (dog flea), *Ctenocephalides felis* (cat flea), *Culicoides* spp. (midges, sandflies, punkies, or no-see-urns), *Damalinia ovis* (sheep biting louse), *Dermatobia* spp. (warble fly), *Gasterophilus haemorrhoidalis* (nose bot fly), *Gasterophilus intestinalis* (common horse bot fly), *Gasterophilus nasalis* (chin fly), *Glossina* spp. (tsetse fly), *Haematobia irritans* (horn fly, buffalo fly), *Haematopinus asini* (horse sucking louse), *Haematopinus eurysternus* (short nosed cattle louse), *Haematopinus ovillus* (body louse), *Haematopinus suis* (hog louse), *Hydrotaea irritans* (head fly), *Hypoderma bovis* (bomb fly), *Hypoderma lineatum* (heel fly), *Linognathus ovillus* (body louse), *Linognathus pedalis* (foot louse), *Linognathus vituli* (long nosed cattle louse), *Lucilia* spp. (maggot fly), *Melophagus ovinus* (sheep ked), *Musca* spp. (house fly, face fly), *Oestrus ovis* (nose bot fly), *Pediculus* spp. (lice), *Phlebotomus* spp. (sandfly), *Phormia regina* (blowfly), *Psorophora* spp. (mosquito), *Pthirus* spp. (lice), *Reduvius* spp. (assassin bug), *Simulium* spp. (black fly), *Solenopotes capillatus* (little blue cattle louse), *Stomoxys calcitrans* (stable fly), *Tabanus* spp. (horse fly), *Tenebrio* spp. (mealworms), *Triatoma* spp. (kissing bugs).

In one preferred embodiment, the present invention is directed to a method for inhibiting a susceptible insect of the order Lepidoptera that comprises applying to a plant an effective insect-inactivating amount of a Formula (1B) compound in accordance with the present invention. Another preferred embodiment of the invention is directed to a method of inhibiting biting flies of the order *Diptera* in animals which comprises administering an effective pest-inhibiting amount of a Formula (1B) compound orally, parenterally, or topically to the animal. Another preferred embodiment of the invention is directed to a method of inhibiting mites of the order *Acarina* which comprises applying to the locus of the mite a mite-inactivating amount of a Formula (1B) compound.

The acid addition salts of the compounds disclosed herein, where possible, are also useful in producing agricultural products. These salts are useful, for example, in separating and purifying the Formula (1A) compounds. In addition, some of these salts may have increased water-solubility. Acid addition salts may be prepared from the compounds disclosed in Formula (1A) where R⁴ is a basic nitrogen-containing sugar molecule such as forosamine. The salts of the compounds are prepared using standard technology for preparing salts which are well known to those skilled in the art. Acid addition salts that are particularly useful include, but are not limited to, salts formed by standard reactions with both organic and inorganic acids such as sulfuric, hydrochloric, phosphoric, acetic, succinic, citric, lactic, maleic, fumaric, cholic, pamoic, mucic, glutamic, camphoric, glutaric, glycolic, phthalic, tartaric, formic, lauric, stearic, salicylic, methanesulfonic, benzenesulfonic, sorbic, picric, benzoic, cinnamic and like acids.

### Insect Screens

### Tobacco Budworm (Heliothis virescens) and Beet Armyworm (Spodoptera littoralis) feeding bioassay.

To compare insecticidal activities of the test compounds against egg and larval stage *Heliothis virescens* or *Spodoptera littoralis,* 50 ul of a 1:1 acetone:water solution was added to each sample and then solublized for one-hour using an orbital shaker. Each of the resulting sample solutions (50, 120, or 250 ppm) was then applied to a well in a 96-well microtiter plate, each well containing an artificial agar-based lepidoptera diet (modified Shorey diet, Southland Products, Lake Village, AR). Each sample was run in triplicate. Plates were allowed to dry for about 4 hours, covered and held for infestation the next day. Each well of the treated plate was infested with 3-5 eggs (0-2 days old) of either *H*. *virescens or S. littoralis.* Plates were then covered with cotton batting and sealed with microplate lid. The treated plates were held inverted in an incubator (80+% rh) for six to seven days. Following the incubation period, the plates were graded for percent mortality using a binocular microscope. Data for each sample was reported as the average percent mortality of the three replicates. Results for selected compounds are reported in Table XI.

**TABLE XI**

| | Beet Armyworm | | | Tobacco Budworm | |
|---|---|---|---|---|---|
| | Diet (6 day) | | | Diet (6 day) | |
| | | Mortality (%) | | | Mortality (%) |
| Compound | Rate (ppm) | | | Rate (ppm) | |
| 1 | 50 | 100 | | 50 | 100 |
| 5 | 50 | 100 | | 50 | 100 |
| 6 | 50 | 100 | | 50 | 100 |
| 32 | 50 | 100 | | 50 | 100 |
| 33 | 50 | 100 | | 50 | 100 |
| 34 | 50 | 100 | | 50 | 100 |
| 37 | 50 | 100 | | 50 | 100 |
| 40 | 50 | 93 | | 50 | 100 |
| 41 | 50 | 100 | | 50 | 100 |
| 43 | 50 | 80 | | 50 | 33 |
| 44 | 50 | 100 | | 50 | 100 |
| 137 | 120 | 100 | | 120 | 100 |
| 141 | 120 | 67 | | 120 | 100 |
| 163 | 120 | 100 | | 120 | 100 |

### Insecticidal Compositions

The Formula (1B) compounds are applied in the form of compositions, which are also a part of this invention. These compositions comprise an insect- or mite-inactivating amount of a Formula (1B) compound in a phytologically acceptable inert carrier. The active component, the Formula (1B) compound, may be present as a single Formula (1B) compound, a mixture of two or more Formula (1B) compounds or a mixture of any of the Formula (1B) compounds together with the dried portion of the fermentation medium in which it is produced.

Compositions are prepared according to the procedures and formulas which are conventional in the agricultural or pest control art, but which are novel and important because of the presence of one or more of the compounds of this invention. The compositions may be concentrated formulations, which are dispersed in water or may be in the form of a dust, bait or granular formulation used without further treatment.

The dispersions in which the compounds or crude dried material are applied are typically aqueous suspensions or emulsions prepared from concentrated formulations of the compounds or crude material. The water-soluble or water-suspension or emulsifiable formulations are either solids, wettable powders, or liquids, known as emulsifiable concentrates or aqueous suspensions. Wettable powders may be agglomerated or compacted to form water dispersible granules. These granules comprise mixtures of compound or crude dried material, inert carriers and surfactants. The concentration of the compound or crude dried material is typically between about 0.1 % to about 90% by weight. The inert carrier is typically attapulgite clays, montmorillonite clays and the diatomaceous earths or purified silicates.

Surfactants comprise typically about 0.5% to about 10% of the wettable powder, where the surfactants are typically sulfonated lignins, condensed napthalene-sulfonates, the napthalene-sulfonates, alkyl-benenesulfonates, alkysulfonates or nonionic surfactants such as ethylene oxide adducts of alkylphenols or mixtures thereof.

Emulsifiable concentrates of the claimed compounds typically range from about 50 to about 500 grams of compound per liter of liquid, equivalent to about 10% to about 50%, dissolved in an inert carrier which is a mixture of a water immiscible solvent and emulsifiers. Organic solvents include organics such as xylenes, and petroleum fractions such as high-boiling naphthlenic and olefinic portions of petroleum which include heavy and aromatic naphtha. Other organics may also be used such as terpenic solvents -rosin derivatives, aliphatic ketones such as cyclohexanone and complex alcohols. Emulsifiers for emulsifiable concentrates are typically mixed ionic and/or nonionic surfactants such as those mentioned herein or their equivalents.

Aqueous suspensions may be prepared containing water-insoluble compounds of this invention, where the compounds are dispersed in an aqueous vehicle at a concentration typically in the range of between about 5% to about 50% by weight. The suspensions are prepared by finely grinding the compound and vigorously mixing it into a vehicle of water, surfactants, and dispersants as discussed herein. Inert ingredients such as inorganic salts and synthetic or natural gums may also be employed to increase the density and/or viscosity of the aqueous vehicle as is desired.

Precipitated flowables may be prepared by dissolving the active molecule in a water-miscible solvent and surfactants or surface-active polymers. When these formulations are mixed with water, the active compound precipitates with the surfactant controlling the size of the resulting micro-crystalline precipitate. The size of the crystal can be controlled through the selection of specific polymer and surfactant mixtures.

The compounds may also be applied as a granular composition that is applied to the soil. The granular composition typically contains from about 0.5% to about 10% by weight of the Formula (1B) compound. The compound is dispersed in an inert carrier which is typically clay or an equivalent substance. Generally, granular compositions are prepared by dissolving the compound in a suitable solvent and applying it to a granular carrier which has been preformed to the desirable particle size. The particle size is typically between about 0.5 mm to 3 mm. The granular compositions may also be prepared by forming a dough or paste of the carrier and compound, drying the combined mixture, and crushing the dough or paste to the desired particle size.

The compounds may also be combined with an appropriate organic solvent. The organic solvent is typically a bland petroleum oil that is widely used in the agricultural industry. These combinations are typically used as a spray. More typically, the compounds are applied as a dispersion in a liquid carrier, where the liquid carrier is water. The compounds may also be applied in the form of an aerosol composition. The compound is dissolved in an inert carrier, which is a pressure-generating propellant mixture. The aerosol composition is packaged in a container, where the mixture is dispersed through an atomizing valve. Propellant mixtures contain either low-boiling halocarbons, which may be mixed with organic solvents or aqueous suspensions pressurized with inert gases or gaseous hydrocarbons.

The amount of compound applied to the loci of insects and mites is not critical and can easily be determined by those skilled in the art. Generally, concentrations of from about 10 ppm to about 5,000 ppm of Formula (1B) compounds provide the desired control. For crops such as soybeans and cotton, the rate of application is about 0.01 to about 1 kg/ha, where the compound is applied in a 5 to 50 gal/A spray formulation. The compounds may be applied to any locus inhabited by an insect or mite. Such locus typically is cotton, soybean and vegetable crops, fruit and nut trees, grape vines, houses and ornamental plants.

The action of the compositions according to the invention can be broadened considerably by adding other, for example insecticidally, acaricidally, and/or nematocidally active, ingredients. For example, one or more of the following compounds can suitably be combined with the compounds of the invention: **organophosphorus compounds** such as acephate, azinphosmethyl, cadusafos, chlorethoxyfos, chlorpyrifos, coumaphos, dematon, demeton-S-methyl, diazinon, dichlorvos, dimethoate, EPN, erthoate, ethoprophos, etrimfos, fenamiphos, fenitrothion, fensulfothion, fenthion, fonofos, formothion, fosthiazate, heptenophos, malathion, methamidophos, methyl parathion, mevinphos, monocrotophos, parathion, phorate, phosalone, phosmet, phosphamidon, phosphocarb, phoxim, profenofos, propaphos, propetamphos, prothiofos, pyrimiphos-methyl, pyrimiphos-ethyl, quinalphos, sulprofos; tebupirimphos, temephos, terbufos, tetrachlorvinphos, thiafenox, thiometon, triazophos, and trichlorphon, , **carbamates** such as aldicarb, bendiocarb, benfuracarb, bensultap, BPMC, butoxycarbocim, carbaryl, carbofuran, carbosulfan, cloethocarb, ethiofencarb, fenobucarb, furathiocarb, methiocarb, isoprocarb, methomyl, oxamyl, pirimicarb, promecarb, propoxur, thiodicarb, and thiofurox, **pyrethroids** such as acrinathrin, allethrin, beta-cyfluthrin, bifenthrin, bioresmethrin, cyfluthrin; cyhalothrin; lambda-cyhalothrin; gamma-cyhalothrin, cypermethrin; alpha-cypermethrin; zeta-cypermethrin; deltamethrin, esfenvalerate, fenvalerate, fenfluthrin, fenpropathrin, flucythrinate, flumethrin, fluvalinate, tau-fluvalinate, halfenprox, permethrin, protrifenbute, resmethrin, silafluofen, tefluthrin, tetramethrin, tralomethrin, fish safe pyrethroids for example ethofenprox, natural pyrethrin, tetramethrin, s-bioallethrin, fenfluthrin and prahethrin, **acylureas, other types of insect growth regulators and insect hormone analogs** such as buprofezin, chromfenozide, chlorfluazuron, diflubenzuron, fenoxycarb, flufenoxuron, halofenozide, hexaflumuron, hydroprene, leufenuron, methoprene, methoxyfenozide, novaluron, pyriproxyfen, teflubenzuron and tebufenozide, N-[3,5-dichloro-2-fluoro-4-(1,1,2,3,3,3-hexafluoropropoxy)phenyl]-N'(2,6-difluorobenzoyl)urea, **neonicotnioids and other nicotinics** such as acetamiprid, AKD-1022, cartap, TI-435, clothiamidin, MTI-446, dinotefuran, imidacloprid, nicotine, nitenpyram, thiamethoxam, thiacloprid, **macrolides** such as avermectins, milbemycins,or spinosyns for example such as abamectin, ivermectin, milbemycin, emamectin benzoate and spinosad, **and other insecticidal, acaricidal, mollscicial and nematocidal compounds or actives** such as aldrin, amitraz, azadirachtin, azocyclotin. bifenazate, bromopropylate, chlordimeform, chlorfenapyr, chlofentezine, chlorobenzilate, chlordane, cyhexatin, cyromazin, DDT, dicofol, dieldrin, DNOC, endosulfan, ethoxazole, fenazaquin, fenbutatin oxide, fenproximate, beta-fenpyroximate, fipronil, flubenzimine, hexythiazox, IKI-220, indoxacarb, lindane, methiocarb, metaldehyde, methoxychlor, neem, petroleum and vegetable oils, pyridaben, pymetrozine, pyrimidifen, rotenone, S-1812, S-9539, spirodiclofen, sulfur, tebufenpyrad, tetradifon, triazamate, an insect-active extract from a plant; a preparation containing insect-active nematodes, a preparation obtainable from *Bacillus subtilis, Bacillus thuringiensis,* a nuclear polyhedrosis virus, or other like organism genetically modified or native, as well as synergists such as piperonyl butoxide, sesamax, safroxan and dodecyl imidazole, and phagostimulants such as cucurbitacin, sugars and Coax.

WO 00/56156 on "Synergistic Insecticide Mixtures" discloses use of certain previously known spinosyn compounds in combination with agonists or antagonists of nicotinic acetylcholine receptors to control animal pests. Particularly preferred examples of such compounds are imidacloprid, acetamiprid, thiamethoxam, nitenpyram, clothiamidin, dinotefuran, thiaclopyrid, and the compound

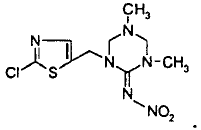

Mixtures of the foregoing agonists and antagonists of nicotinic acetylcholine receptors with the butenyl spinosyn compounds are similarly useful to control animal pests.

WO 00/35282 on "Combination of Active Ingredients" discloses use of spinosad in combination with A) a fungicidally active compound from the series consisting of benomyl, thiophanate-methyl, acibenzolar, flutolanil, furametpyr, fumoxadone, mealaxyl, mefluoxam, azooxystrobin, metominostrobin, capropamide, dicyclocymet, tricyclazole, and oryzemate, and B) an insecticidally active compound from the series consisting of 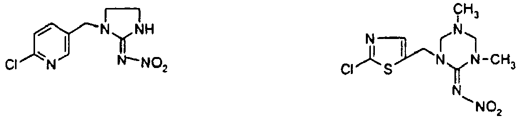 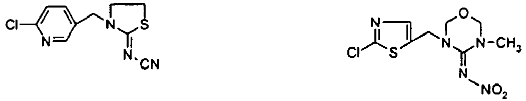 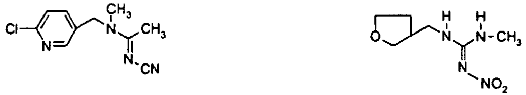 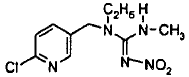 to control insects and fungi. Analogous mixtures in which a butenyl spinosyn compound is substituted for spinosad are similarly useful control insects and fungi.

WO 00/35286 on "Combinations of Active Ingredients" discloses use of a combination of spinosad with A) a compound selected from the group consisting of 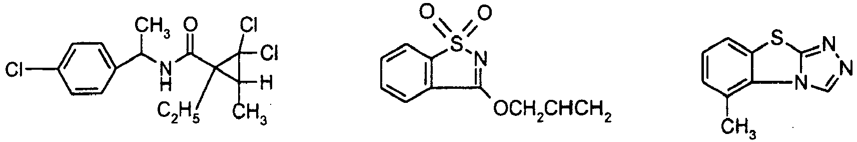 and B) 1-[(6-chloro-3-pyridinyl)-methyl]-N-nitro-2-imidazolidinimine to control animal pests and fungi. Analogous mixtures in which a butenyl spinosyn compound is substituted for spinosad are similarly useful control animal pests and fungi.

WO 99/60856 on "Use of Spinosynes as Soil Insecticides" discloses use of certain previously known spinosyns for treating seeds and for application to plants via the soil or by irrigation to control insects. The butenyl spinosyn compounds can similarly be used for treating seeds and for application to plants via the soil or by irrigation to control insects.

WO 99/33343 on "Use of Macrolides in Pest Control" discloses use of spinosyns to control pests in transgenic crops, use of spinosyns to protect plant propagation material and plant organs formed at a later time from attack by pests. and use of spinosyns to control wood pests and molluscs. The butenyl-spinosyn compounds of the present invention can also be used for these purposes.

### Animal Health Utility

The compounds of the present invention are also useful for the treatment of animals to control arthropods, i.e., insects and arachnids, which are pests on animals. These arthropod pests typically attack their hosts on the external ("ecto") surface; agents which control such pests are referred to as "ectoparasiticides". All animals are subject to attack by such pests, though the problems are most severe among vertebrate hosts. Human beings are potential hosts for many parasites, and in tropical areas and in areas with minimal sanitation, parasitic infections are a regular problem in medical practice. Also highly subject to attack by parasites are the numerous livestock animals, such as cattle, sheep, pigs, goats, buffalo, water buffalo, deer, rabbits, chickens, turkeys, ducks, geese, ostriches, and the like. Horses and other pleasure animals are subject to parasitic attack, as are mink and other animals grown for their fur, and rats, mice and other animals used in laboratory and research settings. Companion animals such as dogs and cats are highly subject to attack by parasites, and because of their close relationship with humans, such parasitism poses problems for the humans with whom they are associated. Fish, crustacea, and other aquatic species are also subject to parasitic attack. In short, parasitism involves as hosts essentially the whole range of animals.

The economic toll from ectoparasitic infestations is large. In the livestock realm, animals suffer reduced feed efficiency and growth rates. Milk and wool production suffer, and there is damage to fleece, hides, and pelts. Animals are rendered susceptible to secondary microbiological infections and to further parasite attack. Ectoparasites also cause considerable discomfort even when they are not severely detrimental to health and production.

Although a number of parasiticides are in use, they suffer from a variety of problems, including a limited spectrum of activity, environmental toxicity, the need for repeated treatment, and, in many instances, resistance by ectoparasites. Therefore, there is a continuing need for new ectoparasiticides.

The compounds of Formula (1B) provide a new tool in the armamentarium for controlling ectoparasites. In this embodiment, the present invention is directed to a method for inhibiting or killing an arthropod pest on a host animal, which comprises contacting the pest with an effective amount of a compound of the present invention.

The compounds of Formula (1B) can be used to control a wide variety of arthropod pests including various flies and fly larvae, fleas, lice, mites, and ticks. The compounds' ectoparasiticidal activity is achieved when the compounds contact the pests. The contact can be of the egg, larvae, adult, or other life stage. "Contact" includes ingestion of the compound by the pest.

Techniques for delivering ectoparasiticides are well known to those skilled in the art. In general, a present compound is applied to the exterior surface of an animal, whereby it contacts pests already present on the host as well as those which arrive on the host's body within the efficacy period of the compound. Typically, the compound is formulated in a liquid formulation which is sprayed onto the animal's surface or poured onto the animal's surface. Another conventional treatment is a "dip", whereby cattle are treated by being substantially immersed in a dilute solution of an ectoparasiticide. For some hosts and pests, the formulation can be a dust, which is sprinkled onto the host, or a shampoo or cream which is employed in bathing the animal. Collars on cats and dogs are also employed as a way of delivering an ectoparasiticide directly to the animal's surface.

In another embodiment, the compounds of the invention can be delivered to animals using ear tags, a delivery method disclosed in US 4,265,876.

In another technique, an ectoparasiticide is applied to locations frequented by animals, so that pests are thereby contacted by the compound even as in direct application to the host. Application to pet bedding is well known, as is application to carpeting. For cattle, dusting bags are well known. These are positioned in a doorway where the cattle inevitably rub against the bag and pests are contacted by the present compound.

In yet another embodiment, the present compounds can be used to control insects and arachnids which are pests in the feces of cattle and other animals. In this embodiment, the compounds are administered orally and the compounds travel through the intestinal tract and emerge in the feces. Control of pests in the feces indirectly protects the animals from the pests.

The compounds are formulated for use as ectoparasiticides in manners known to those skilled in the art. In general, a formulation will include a compound of the present invention and one or more physiologically acceptable adjuvants. Formulations include concentrated versions, in which the present active agent is present in a concentration of from 0.001 to 98.0 percent, with the remaining content being physiologically acceptable carriers. Such formulations, especially those with less than 50 percent of the present compound, can sometimes be used directly, but these formulations can also be diluted with other physiologically acceptable carriers to form more dilute treating formulations. These latter formulations can include the active agent in lesser concentrations of from 0.001 to 0.1 percent.

### Human Pharmaceutical Utility

The compounds of the invention are also useful as human pharmaceuticals to control parasites, for example, lice. The compounds can be used, for example, in the formulations for controlling lice that are disclosed in WO 00/01347.

The Anoplura, or sucking lice, are parasites found on nearly all groups of mammals. Of the 15 recognized families of Anoplura, two families, *Pediculidae* and *Pthiridae,* have species found on humans. *Pediculus humanus* is the only species in the family *Pediculidae* that infests humans. It includes the head louse, *Pediculus humanus capitis*; and the body or clothing louse, *Pediculus humanus humanus,* sometimes called *Pediculus corporis.* The crab louse, *Pthirus pubis,* is a distinct species and is the only member of the family *Pthiridae* that infests humans. As used herein, the term "human lice or louse" includes a member *of Pediculus humanus* or *Pthirus pubis.*

Accordingly, in one of its aspects, the invention provides pediculicidal/ovicidal (anti-lice) formulations for controlling a lice infestation in a human comprising as an active ingredient a spinosyn, or a physiologically acceptable derivative or salt thereof, and a physiologically acceptable carrier. Especially useful formulations of this invention are hair-care formulations. Especially useful hair-care formulations are shampoos. The invention also provides methods of using these formulations to control human lice species. These formulations and methods control lice in a safer, more effective manner than previously known anti-lice formulations and methods.

The anti-lice formulations of this invention may be formulated in a number of ways. Particularly useful formulations are shampoos, conditioners, and lotions of the type disclosed in WO 00/01347.

When used in a shampoo formulation, hair conditioner formulation, or lotion the spinosyn component is present at a level of from about 0.1% to about 30%, preferably from about 1% to about 10%.

Specific embodiments contemplated include:
A. A formulation for controlling a lice infestation in a human comprising as an active ingredient a compound of formula 1 or 2 where R5 is a group having one of the formulas 4a through 4i, or a physiologically acceptable derivative or salt thereof, and a physiologically acceptable carrier.
B. A formulation of embodiment A that is a hair care formulation.
C. A pediculicidal shampoo comprising:
   (a) from about 0.1 % to about 30% of a compound of formula 1 or 2 where R5 is a group having one of the formulas 4a through 4i, or a physiologically acceptable derivative or salt thereof;
   (b) from about 5% to about 30% of a synthetic surfactant;
   (c) from about 1% to about 7% of an amide; and
   (d) water.
D. A shampoo of embodiment C wherein the synthetic surfactant is anionic, amphoteric, cationic, zwitterionic, or non-ionic, or a mixture thereof.
E. A shampoo of embodiment D wherein the amide is coconut monoethanolamide, coconut diethanolamide or a mixture thereof.
F. A shampoo of embodiment D additionally comprising from about 1% to about 10% of a non-volatile silicone material.
G. A shampoo of embodiment F wherein the non-volatile silicone is a polyalkyl siloxane, polyalkylaryl siloxane, polyether siloxane co-polymer, or a mixture thereof, whose viscosity is from about 100 centipoise to about 150,000,000 centipoise at 25□.
H. A shampoo of embodiment G additionally comprising from about 0.5% to about 5% of a suspending agent selected from the group consisting of crystalline amphiphilic materials having needle-like or platelet structures, polymeric materials, clays, fumed metal oxides, and mixtures thereof.
I. A shampoo of embodiment H wherein the suspending agent is a crystalline amphiphilic material selected from the group consisting of long chain C₁₆-C₂₂ acyl derivatives, long chain C₁₆-C₂₂ alkanolamide of fatty acids, and mixtures thereof.
J. A shampoo of embodiment I wherein the suspending agent is an ethylene glycol diester.
K. A shampoo of embodiment D wherein the amount of a spinosyn, or derivative or salt thereof, is at a level from about 0.25% to about 1.5%.
L. A method for controlling a lice infestation in a human comprising topically administering a formulation of embodiment A to the human.
M. The method of embodiment L wherein the lice infestation is *Pediculus humanus capitis.*
N. The method of embodiment L wherein the lice infestation is *Pediculus humanus humanus.*
O. The method of embodiment L wherein the lice infestation is *Pthirus pubis.*
P. A method for treating human hair to kill and facilitate removal of lice and their eggs comprising the steps of:
   (a) applying from about 10 g to about 30 g of a formulation comprising a compound of formula 1 or 2, where R5 is a group having one of the formulas 4a through 4i, or a physiologically acceptable derivative or salt thereof, and a physicologically acceptable carrier to wet hair;
   (b) working the formulation through the hair and scalp;
   (c) leaving the formulation on the hair and scalp for about 6-10 minutes;
   (d) removing the formulation from the hair by rinsing, with water.
Q. The use of a compound of formula 1 or 2, where R5 is a group having one of the formulas 4a through 4i, or a physiologically acceptable derivative or salt thereof, or a formulation containing either entity, for the manufacture of a medicament for controlling lice in a human.

## Claims

1. A compound of formula (1A) or (2A) 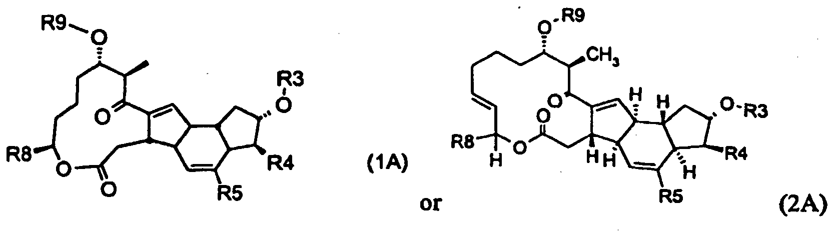 wherein:
R³ is a group selected from 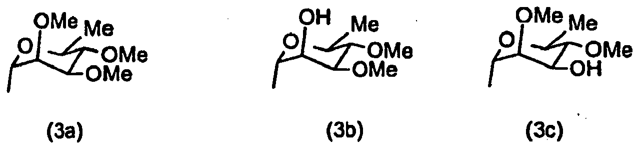 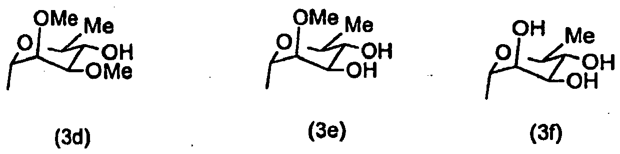
R⁴ is a hydrogen atom or a hydroxyl group,
R⁵ is a hydrogen atom or methyl group,
R⁸ is 1-butenyl, 1,3-butadienyl, n-butyl, 3-hydroxy-1-butenyl, butan-3-one, 1,2-epoxy-butyl or 1-propenyl,
R⁹ is a hydrogen atom or a group selected from 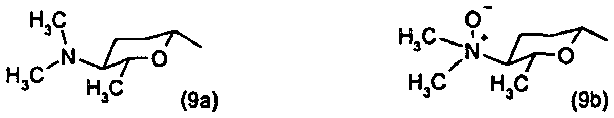 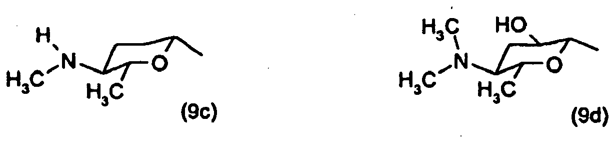 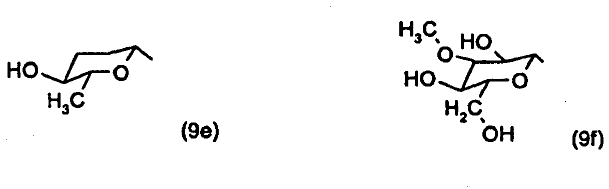 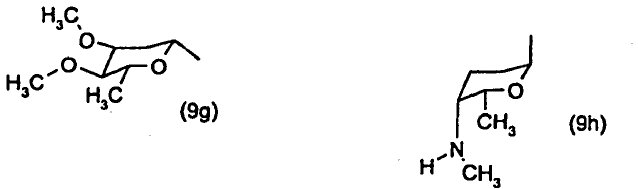 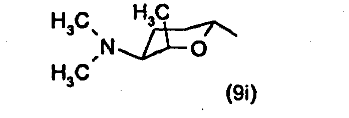
provided that:
a) if R³ is a group of formula (3b) or (3c), R⁴ and R⁵ are H, and R⁸ is 1-butenyl, then R⁹ is neither H nor a group of formula (9a); and
b) if R³ is a group of formula (3a), then R⁴ and R⁵ are H, R⁸ is 1-propenyl, and R⁹ is H.

2. A compound of claim 1 wherein R⁸ is 1-butenyl, 1,3-butadienyl, n-butyl, 3-hydroxy-1-butenyl, or 1-propenyl.

3. A compound of claim 1 wherein R³, R⁴, R⁵, R⁸, and R⁹ are present in one of the following combinations:
| cmpd. no. | formula | R³ | R⁴ | R⁵ | R⁸ | R⁹ |
|---|---|---|---|---|---|---|
| 32 | (1A) | (3d) | H | H | 1-butenyl | (9a) |
| 33 | (1A) | (3f) | H | H | 1-butenyl | (9a) |
| 34 | (1A) | (3d) | OH | H | 1-butenyl | (9a) |
| 35 | (1A) | (3d) | H | H | 1-butenyl | (9c) |
| 36 | (1A) | (3d) | H | CH₃ | 1-butenyl | (9a) |
| 37 | (1A) | (3e) | H | H | 1-butenyl | (9a) |
| 38 | (1A) | (3a) | H | H | 1-propenyl | H |
| 39 | (1A) | (3d) | H | H | 1-butenyl | H |
| 40 | (1A) | (3e) | OH | H | 1-butenyl | (9a) |
| 41 | (1A) | (3c) | OH | H | 1-butenyl | (9a) |
| 42 | (1A) | (3e) | H | H | 1-butenyl | (9b) |
| 43 | (1A) | (3e) | H | H | 1-propenyl | (9a) |
| 44 | (1A) | (3e) | H | CH₃ | 1-butenyl | (9a) |
| 45 | (1A) | (3d) | H | H | 1-butenyl | (9c) |
| 46 | (1A) | (3d) | H | H | 1-butenyl | (9d) |
| 47 | (1A) | (3d) | H | H | 3-hydroxy-1-butenyl | (9a) |
| 48 | (1A) | (3d) | H | H | 3-hydroxy-1-butenyl | (9c) |
| 49 | (1A) | (3d) | H | H | 1,3-butadienyl | (9a) |
| 50 | (1A) | (3d) | H | H | 1-butenyl | (9e) |
| 51 | (1A) | (3d) | H | H | 1-butenyl | (9f) |
| 52 | (1A) | (3d) | OH | H | 1-butenyl | (9e) |
| 53 | (1A) | (3d) | OH | H | 1-butenyl | (9g) |
| 54 | (1A) | (3d) | H | H | 1-propenyl | (9a) |
| 55 | (1A) | (3d) | H | CH₃ | 1-butenyl | H |
| 56 | (1A) | (3d) | OH | H | 1-butenyl | H |
| 57 | (1A) | (3d) | H | H | 3-hydroxy-1-butenyl | H |
| 58 | (1A) | (3d) | OH | H | 3-hydroxy-1-butenyl | H |
| 59 | (1A) | (3d) | H | H | 1,3-butadienyl | H |
| 60 | (1A) | (3d) | H | H | n-butyl | H |
| 61 | (1A) | (3d) | H | H | 1-butenyl | (9h) |
| 62 | (1A) | (3d) | H | H | 1-butenyl | (9i) |
| 63 | (1A) | (3d) | OH | H | 1,3-butadienyl | (9a) |
| 64 | (1A) | (3d) | OH | CH₃ | 1-butenyl | (9a) |
| 65 | (1A) | (3d) | OH | H | 1-propenyl | (9a) |
| 66 | (1A) | (3e) | H | H | 1-butenyl | (9c) |
| 67 | (1A) | (3e) | H | H | 1-butenyl | (9d) |
| 68 | (1A) | (3e) | H | H | 3-hydroxy-1-butenyl | (9a) |
| 69 | (1A) | (3c) | H | H | 3-hydroxy-1-butenyl | (9c) |
| 70 | (1A) | (3e) | H | H | 1,3-butadienyl | (9a) |
| 71 | (1A) | (3e) | H | H | 1-butenyl | (9e) |
| 72 | (1A) | (3e) | H | H | 1-butenyl | (9f) |
| 73 | (1A) | (3e) | OH | H | 1-butenyl | (9e) |
| 74 | (1A) | (3e) | OH | H | 1-butenyl | (9g) |
| 75 | (1A) | (3e) | H | H | 1-butenyl | H |
| 76 | (1A) | (3e) | H | CH₃ | 1-butenyl | H |
| 77 | (1A) | (3e) | OH | H | 1-butenyl | H |
| 78 | (1A) | (3e) | H | H | 3-hydroxyl-1-butenyl | H |
| 79 | (1A) | (3e) | OH | H | 3-hydroxy-1-butenyl | H |
| 80 | (1A) | (3e) | H | H | 1,3-butadienyl | H |
| 81 | (1A) | (3e) | H | H | n-butyl | H |
| 82 | (1A) | (3e) | H . | H | 1-butenyl | (9h) |
| 83 | (1A) | (3e) | H | H | 1-butenyl | (9i) |
| 84 | (1A) | (3e) | OH | H | 1,3-butadienyl | (9a) |
| 85 | (1A) | (3e) | OH | CH₃ | 1-butenyl | (9a) |
| 86 | (1A) | (3e) | OH | H | 1-propenyl | (9a) |
| 87 | (1A) | (3f) | H | H | 1-butenyl | (9b) |
| 88 | (1A) | (3f) | H | H | 1-butenyl | (9c) |
| 89 | (1A) | (3f) | H | H | 1-butenyl | (9d) |
| 90 | (1A) | (3f) | H | CH₃ | 1-butenyl | (9a) |
| 91 | (1A) | (3f) | OH | H | 1-butenyl | (9a) |
| 92 | (1A) | (3f) | H | H | 3-hydroxy-1-butenyl | (9a) |
| 93 | (1A) | (3f) | H | H | 3-hydroxy-1-butenyl | (9c) |
| 94 | (1A) | (3f) | H | H | 1,3-butadienyl | (9a) |
| 95 | (1A) | (3f) | H | H | 1-butenyl | (9e) |
| 96 | (1A) | (3f) | H | H | 1-butenyl | (9f) |
| 97 | (1A) | (3f) | OH | H | 1-butenyl | (9e) |
| 98 | (1A) | (3f) | OH | H | 1-butenyl | (9g) |
| 99 | (1A) | (3f) | H | H | 1-propenyl | (9a) |
| 100 | (1A) | (3f) | H | H | 1-butenyl | H |
| 101 | (1A) | (3f) | H | CH₃ | 1-butenyl | H |
| 102 | (1A) | (3f) | OH | H | 1-butenyl | H |
| 103 | (1A) | (3f) | H | H | 3-hydroxy-1-butenyl | H |
| 104 | (1A) | (3f) | OH | H | 3-hydroxy-1-butenyl | H |
| 105 | (1A) | (3f) | H | H | 1,3-butadienyl | H |
| 106 | (1A) | (3f) | H | H | n-butyl | H |
| 107 | (1A) | (3f) | H | H | 1-butenyl | (9h) |
| 108 | (1A) | (3f) | H | H | 1-butenyl | (9i) |
| 109 | (1A) | (3f) | OH | H | 1,3-butadienyl | (9a) |
| 110 | (1A) | (3f) | OH | CH₃ | 1-butenyl | (9a) |
| 111 | (1A) | (3f) | OH | H | 1-propenyl | (9a) |
| 112 | (1A) | (3b) | H | H | 1-butenyl | (9b) |
| 113 | (1A) | (3b) | H | H | 1-butenyl | (9c) |
| 114 | (1A) | (3b) | H | H | 1-butenyl | (9d) |
| 115 | (1A) | (3b) | H | CH₃ | 1-butenyl | (9a) |
| 116 | (1A) | (3b) | OH | H | 1-butenyl | (9a) |
| 117 | (1A) | (3b) | H | H | 3-hydroxy-1-butenyl | (9a) |
| 118 | (1A) | (3b) | H | H | 3-hydroxy-1-butenyl | (9c) |
| 119 | (1A) | (3b) | H | H | 1,3-butadienyl | (9a) |
| 120 | (1A) | (3b) | H | H | 1-butenyl | (9e) |
| 121 | (1A) | (3b) | H | H | 1-butenyl | (9f) |
| 122 | (1A) | (3b) | OH | H | 1-butenyl | (9e) |
| 123 | (1A) | (3b) | OH | H | 1-butenyl | (9g) |
| 124 | (1A) | (3b) | H | H | 1-propenyl | (9a) |
| 125 | (1A) | (3b) | H | CH₃ | 1-butenyl | H |
| 126 | (1A) | (3b) | OH | H | 1-butenyl | H |
| 127 | (1A) | (3b) | H | H | 3-hydroxy-1-butenyl | H |
| 128 | (1A) | (3b) | OH | H | 3-hydroxy-1-butenyl | H |
| 129 | (1A) | (3b) | H | H | 1,3-butadienyl | H |
| 130 | (1A) | (3b) | H | H | n-butyl | H |
| 131 | (1A) | (3b) | H | H | 1-butenyl | (9h) |
| 132 | (1A) | (3b) | H | H | 1-butenyl | (9i) |
| 133 | (1A) | (3b) | OH | H | 1,3-butadienyl | (9a) |
| 134 | (1A) | (3b) | OH | CH₃ | 1-butenyl | (9a) |
| 135 | (1A) | (3b) | OH | H | 1-propenyl | (9a) |
| 136 | (1A) | (3c) | H | H | 1-butenyl | (9b) |
| 137 | (1A) | (3c) | H | H | 1-butenyl | (9c) |
| 138 | (1A) | (3c) | H | H | 1-butenyl | (9d) |
| 139 | (1A) | (3c) | H | CH₃ | 1-butenyl | (9a) |
| 140 | (1A) | (3c) | OH | H | 1-butenyl | (9a) |
| 141 | (1A) | (3c) | H | H | 3-hydroxy-1-butenyl | (9a) |
| 142 | (1A) | (3c) | H | H | 3-hydroxy-1-butenyl | (9c) |
| 143 | (1A) | (3c) | H | H | 1,3-butadienyl | (9a) |
| 144 | (1A) | (3c) | H | H | 1-butenyl | (9e) |
| 145 | (1A) | (3c) | H | H | 1-butenyl | |
| 146 | (1A) | (3c) | OH | H | 1-butenyl | (9e) |
| 147 | (1A) | (3c) | OH | H | 1-butenyl | (9g) |
| 148 | (1A) | (3c) | H | H | 1-propenyl | (9a) |
| 149 | (1A) | (3c) | H | CH₃ | 1-butenyl | H |
| 150 | (1A) | (3c) | OH | H | 1-butenyl | H |
| 151 | (1A) | (3c) | H | H | 3-hydroxy-1-butenyl | H |
| 152 | (1A) | (3c) | OH | H | 3-hydroxy-1-butenyl | H |
| 153 | (1A) | (3c) | H | H | 1,3-butadienyl | H |
| 154 | (1A) | (3c) | H | H | n-butyl | H |
| 155 | (1A) | (3c) | H | H | 1-butenyl | (9h) |
| 156 | (1A) | (3c) | H | H | 1-butenyl | (9i) |
| 157 | (1A) | (3c) | OH | H | 1,3-butadienyl | (9a) |
| 158 | (1A) | (3c) | OH | CH₃ | 1-butenyl | (9a) |
| 159 | (1A) | (3c) | OH | H | 1-propenyl | (9a) |
| 165 | (1A) | (3c) | H | H | butan-3-one | (9a) |
| 166 | (1A) | (3c) | H | H | 1,2-epoxy-butyl | (9a) |
| 167 | (1A) | (3d) | H | H | butan-3-one | (9a) |
| 168 | (1A) | (3d) | H | H | 1,2-epoxy-butyl | (9a) |
| 169 | (1A) | (3e) | H | H | butan-3-one | (9a) |
| 170 | (1A) | (3e) | H | H | 1,2-epoxy-butyl | (9a) |
| 171 | (1A) | (3e) | H | H | butan-3-one | (9a) |
| 172 | (1A) | (3e) | H | H | 1,2-epoxy-butyl | (9a) |
| 173 | (1A) | (3b) | H | H | butan-3-one | (9a) |
| 174 | (1A) | (3b) | H | H | 1,2-epoxy-butyl | (9a) |
| 175 | (1A) | (3a) | H | H | butan-3-one | (9a) |
| 176 | (1A) | (3a) | H | H | 1,2-epoxy-butyl | (9a) |
| 177 | (1A) | (3a) | OH | H | butan-3-one | (9a) |
| 178 | (1A) | (3a) | OH | H | 1,2-epoxy-butyl | (9a) |
| 179 | (1A) | (3a) | H | CH₃ | butan-3-one | (9a) |
| 180 | (1A) | (3a) | H | CH₃ | 1,2-epoxy-butyl | (9a) |
| 181 | (1A) | (3a) | OH | CH₃ | butan-3-one | (9a) |
| 182 | (1A) | (3a) | OH | CH₃ | 1,2-epoxy-butyl | (9a) |
| 183 | (1A) | (3b) | H | H | butan-3-one | (9b) |
| 184 | (1A) | (3b) | H | H | butan-3-one | (9c) |
| 185 | (1A) | (3b) | H | H | butan-3-one | (9d) |
| 186 | (1A) | (3b) | H | H | butan-3-one | (9e) |
| 187 | (1A) | (3b) | H | H | butan-3-one | (9f) |
| 188 | (1A) | (3b) | OH | H | butan-3-one | (9e) |
| 189 | (1A) | (3b) | OH | H | butan-3-one | (9g) |
| 190 | (1A) | (3b) | H | CH₃ | butan-3-one | H |
| 191 | (1A) | (3b) | OH | H | butan-3-one | H |
| 192 | (1A) | (3b) | H | H | butan-3-one | (9h) |
| 193 | (1A) | (3b) | H | H | butan-3-one | (9i) |
| 194 | (1A) | (3b) | H | H | 1,2-epoxy-butyl | (9b) |
| 195 | (1A) | (3b) | H | H | 1,2-epoxy-butyl | (9c) |
| 196 | (1A) | (3b) | H | H | 1,2-epoxy-butyl | (9d) |
| 197 | (1A) | (3b) | H | H | 1,2-epoxy-butyl | (9e) |
| 199 | (1A) | (3b) | H | H | 1,2-epoxy-butyl | (9f) |
| 200 | (1A) | (3b) | OH | H | 1,2-epoxy-butyl | (9e) |
| 201 | (1A) | (3b) | OH | H | 1,2-epoxy-butyl | (9g) |
| 202 | (1A) | (3b) | H | CH₃ | 1,2-epoxy-butyl | H |
| 203 | (1A) | (3b) | OH | H | 1,2-epoxy-butyl | H |
| 204 | (1A) | (3b) | H | H | 1,2-epoxy-butyl | (9h) |
| 205 | (1A) | (3b) | H | H | 1,2-epoxy-butyl | (9i) |

4. A compound of claim 1 of the Formula (1A).

5. A compound of claim 4 wherein R³, R⁴, R⁵, R⁸, and R⁹ are present in one of the following combinations:
| cmpd no. | R³ | R⁴ | R⁵ | R⁸ | R⁹ |
|---|---|---|---|---|---|
| 32 | (3d) | H | H | 1-butenyl | (9a) |
| 33 | (3f) | H | H | 1-butenyl | (9a) |
| 34 | (3d) | OH | H | 1-butenyl | (9a) |
| 35 | (3d) | H | H | 1-butenyl | (9c) |
| 36 | (3d) | H | CH₃ | 1-butenyl | (9a) |
| 37 | (3e) | H | H | 1-butenyl | (9a) |
| 38 | (3a) | H | H | 1-propenyl | H |
| 39 | (3d) | H | H | 1-butenyl | H |
| 40 | (3e) | OH | H | 1-butenyl | (9a) |
| 41 | (3c) | OH | H | 1-butenyl | (9a) |
| 42 | (3e) | H | H | 1-butenyl | (9b) |
| 43 | (3e) | H | H | 1-propenyl | (9a) |
| 44 | (3e) | H | CH₃ | 1-butenyl | (9a) |
| 45 | (3d) | H | H | 1-butenyl | (9c) |
| 46 | (3d) | H | H | 1-butenyl | (9d) |
| 47 | (3d) | H | H | 3-hydroxy-1-butenyl | (9a) |
| 48 | (3d) | H | H | 3-hydroxy-1-butenyl | (9c) |
| 49 | (3d) | H | H | 1,3-butadienyl | (9a) |
| 50 | (3d) | H | H | 1-butenyl | (9e) |
| 51 | (3d) | H | H | 1-butenyl | (9f) |
| 52 | (3d) | OH | H | 1-butenyl | (9e) |
| 53 | (3d) | OH | H | 1-butenyl | (9g) |
| 54 | (3d) | H | H | 1-propenyl | (9a) |
| 55 | (3d) | H | CH₃ | 1-butenyl | H |
| 56 | (3d) | OH | H | 1-butenyl | H |
| 57 | (3d) | H | H | 3-hydroxy-1-butenyl | H |
| 58 | (3d) | OH | H | 3-hydroxy-1-butenyl | H |
| 59 | (3d) | H | H | 1,3-butadienyl | H |
| 60 | (3d) | H | H | n-butyl | H |
| 61 | (3d) | H | H | 1-butenyl | (9h) |
| 62 | (3d) | H | H | 1-butenyl | (9i) |
| 63 | (3d) | OH | H | 1,3-butadienyl | (9a) |
| 64 | (3d) | OH | CH₃ | 1-butenyl | (9a) |
| 65 | (3d) | OH | H | 1-propenyl | (9a) |
| 66 | (3e) | H | H | 1-butenyl | (9c) |
| 67 | (3c) | H | H | 1-butenyl | (9d) |
| 68 | (3e) | H | H | 3-hydroxy-1-butenyl | (9a) |
| 69 | (3e) | H | H | 3-hydroxy-1-butenyl | (9c) |
| 70 | (3e) | H | H | 1,3-butadienyl | (9a) |
| 71 | (3e) | H | H | 1-butenyl | (9e) |
| 72 | (3e) | H | H | 1-butenyl | (9f) |
| 73 | (3e) | OH | H | 1-butenyl | (9e) |
| 74 | (3e) | OH | H | 1-butenyl | (9g) |
| 75 | (3e) | H | H | 1-butenyl | H |
| 76 | (3e) | H | CH₃ | 1-butenyl | H |
| 77 | (3e) | OH | H | 1-butenyl | H |
| 78 | (3e) | H | H | 3-hydroxy-1-butenyl | H |
| 79 | (3e) | OH | H | 3-hydroxy-1-butenyl | H |
| 80 | (3e) | H | H | 1,3-butadienyl | H |
| 81 | (3e) | H | H | n-butyl | H |
| 82 | (3e) | H | H | 1-butenyl | (9h) |
| 83 | (3e) | H | H | 1-butenyl | (9i) |
| 84 | (3e) | OH | H | 1,3-butadienyl | (9a) |
| 85 | (3e) | OH | CH₃ | 1-butenyl | (9a) |
| 86 | (3e) | OH | H | 1-propenyl | (9a) |
| 87 | (3f) | H | H | 1-butenyl | (9b) |
| 88 | (3f) | H | H | 1-butenyl | (9c) |
| 89 | (3f) | H | H | 1-butenyl | (9d) |
| 90 | (3f) | H | CH₃ | 1-butenyl | (9a) |
| 91 | (3f) | OH | H | 1-butenyl | (9a) |
| 92 | (3f) | H | H | 3-hydroxy-1-butenyl | (9a) |
| 93 | (3f) | H | H | 3-hydroxy-1-butenyl | (9c) |
| 94 | (3f) | H | H | 1,3-butadienyl | (9a) |
| 95 | (3f) | H | H | 1-butenyl | (9e) |
| 96 | (3f) | H | H | 1-butenyl | (9f) |
| 97 | (3f) | OH | H | 1-butenyl | (9e) |
| 98 | (3f) | OH | H | 1-butenyl | (9g) |
| 99 | (3f) | H | H | 1-propenyl | (9a) |
| 100 | (3f) | H | H | 1-butenyl | H |
| 101 | (3f) | H | CH₃ | 1-butenyl | H |
| 102 | (3f) | OH | H | 1-butenyl | H |
| 103 | (3f) | H | H | 3-hydroxy-1-butenyl | H |
| 104 | (3f) | OH | H | 3-hydroxy-1-butenyl | H |
| 105 | (3f) | H | H | 1,3-butadienyl | H |
| 106 | (3f) | H | H | n-butyl | H |
| 107 | (3f) | H | H | 1-butenyl | (9h) |
| 108 | (3f) | H | H | 1-butenyl | (9i) |
| 109 | (3f) | OH | H | 1,3-butadienyl | (9a) |
| 110 | (3f) | OH | CH₃ | 1-butenyl | (9a) |
| III | (3f) | OH | H | 1-propenyl | (9a) |
| 112 | (3b) | H | H | 1-butenyl | (9b) |
| 113 | (3b) | H | H | 1-butenyl | (9c) |
| 114 | (3b) | H | H | 1-butenyl | (9d) |
| 115 | (3b) | H | CH₃ | 1-butenyl | (9a) |
| 116 | (3b) | OH | H | 1-butenyl | (9a) |
| 117 | (3b) | H | H | 3-hydroxy-1-butenyl | (9a) |
| 118 | (3b) | H | H | 3-hydroxy-1-butenyl | (9c) |
| 119 | (3b) | H | H | 1,3-butadienyl | (9a) |
| 120 | (3b) | H | H | 1-butenyl | (9e) |
| 121 | (3b) | H | H | 1-butenyl | (9f) |
| 122 | (3b) | OH | H | 1-butenyl | (9e) |
| 123 | (3b) | OH | H | 1-butenyl | (9g) |
| 124 | (3b) | H | H | 1-propenyl | (9a) |
| 125 | (3b) | H | CH₃ | 1-butenyl | H |
| 126 | (3b) | OH | H | 1-butenyl | H |
| 127 | (3b) | H | H | 3-hydroxy-1-butenyl | H |
| 128 | (3b) | OH | H | 3-hydroxy-1-butenyl | H |
| 129 | (3b) | H | H | 1,3-butadienyl | H |
| 130 | (3b) | H | H | n-butyl | H |
| 131 | (3b) | H | H | 1-butenyl | (9h) |
| 132 | (3b) | H | H | 1-butenyl | (9i) |
| 133 | (3b) | OH | H | 1,3-butadienyl | (9a) |
| 134 | (3b) | OH | CH₃ | 1-butenyl | (9a) |
| 135 | (3b) | OH | H | 1-propenyl | (9a) |
| 136 | (3c) | H | H | 1-butenyl | (9b) |
| 137 | (3c) | H | H | 1-butenyl | (9c) |
| 138 | (3c) | H | H | 1-butenyl | (9d) |
| 139 | (3c) | H | CH₃ | 1-butenyl | (9a) |
| 140 | (3c) | OH | H | 1-butenyl | (9a) |
| 141 | (3c) | H | H | 3-hydroxy-1-butenyl | (9a) |
| 142 | (3c) | H | H | 3-hydroxy-1-butenyl | (9c) |
| 143 | (3c) | H | H | 1,3-butadienyl | (9a) |
| 144 | (3c) | H | H | 1-butenyl | (9e) |
| 145 | (3c) | H | H | 1-butenyl | (9f) |
| 146 | (3c) | OH | H | 1-butenyl | (9e) |
| 147 | (3c) | OH | H | 1-butenyl | (9g) |
| 148 | (3c) | H | H | 1-propenyl | (9a) |
| 149 | (3c) | H | CH₃ | 1-butenyl | H |
| 150 | (3c) | OH | H | 1-butenyl | H |
| 151 | (3c) | H | H | 3-hydroxy-1-butenyl | H |
| 152 | (3c) | OH | H | 3-hydroxy-1-butenyl | H |
| 153 | (3c) | H | H | 1,3-butadienyl | H |
| 154 | (3c) | H | H | n-butyl | H |
| 155 | (3c) | H | H | 1-butenyl | (9h) |
| 156 | (3c) | H | H | 1-butenyl | (9i) |
| 157 | (3c) | OH | H | 1,3-butadienyl | (9a) |
| 158 | (3c) | OH | CH₃ | 1-butenyl | (9a) |
| 159 | (3c) | OH | H | 1-propenyl | (9a) |

6. A compound of the formula (10):

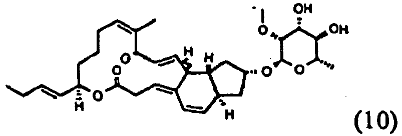

7. A compound of the formula (11)

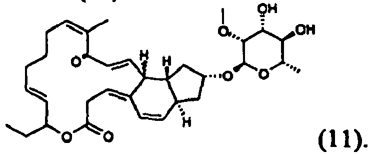

8. A compound of claim 1 wherein R⁹ is other than H.

9. A compound of claim 3 wherein R⁹ is other than H.

10. An insecticide or acaricide composition which comprises an insect, mite, or tick inactivating amount of a compound of claim 8 in combination with a phytologically- or physiologically- acceptable carrier.

11. A compound of claim 8 for use as an insecticide or acaricide.

12. A compound of claim 8 for use in protecting a locus from infestation by insects, mites, or ticks.

13. A compound of claim 8 for use in controlling a population of parasites that infest a host animal.

14. A formulation for use in controlling a lice infestation in a human comprising as an active ingredient a compound of claim 8, or a physiologically acceptable derivative or salt thereof, and a physiologically acceptable carrier.

15. A compound of claim 8 for use in controlling a lice infestation in a human.

16. A process for preparing a compound of claim 1 which comprises culturing a strain selected from
NRRL 30424,
NRRL 30423,
NRRL 30422,
NRRL 30438,
NRRL 30421, and
NRRL 30437,
or a strain derived therefrom that produces a compound of claim 1, in a suitable culture medium under submerged aerobic fermentation conditions until a recoverable amount of a compound of claim 1 is produced, and recovering a compound of claim 1 from the culture medium.

17. A biologically pure culture of NRRL 30424
or a strain derived therefrom that produces a compound of claim 1.

18. A biologically pure culture of NRRL 30423
3 or a strain derived therefrom that produces a compound of claim 1.

19. A biologically pure culture of NRRL 30422
or a strain derived therefrom that produces a compound of claim 1.

20. A biologically pure culture of NRRL 30438
or a strain derived therefrom that produces a compound of claim 1.

21. A biologically pure culture of NRRL 30421
or a strain derived therefrom that produces a compound of claim 1.

22. A biologically pure culture of NRRL 30437
or a strain derived therefrom that produces a compound of claim 1.

## Patentansprüche

1. Eine Verbindung der Formel (1A) oder (2A) 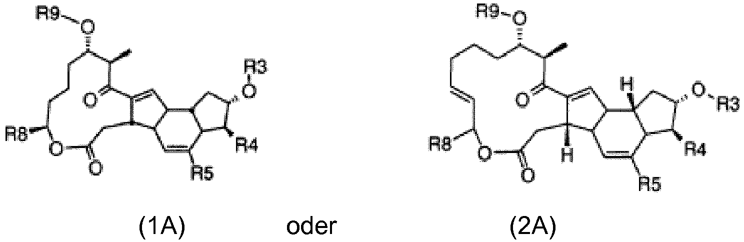 worin:
R³ eine Gruppe ist, ausgewählt aus 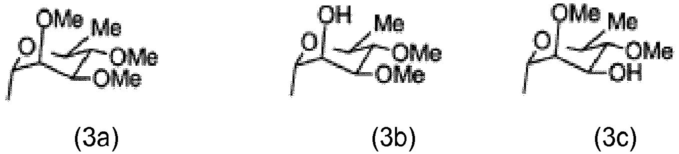 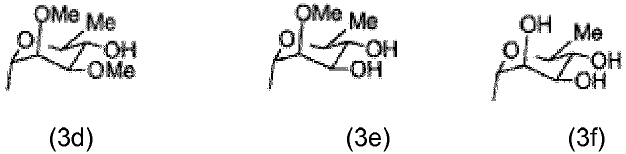
R⁴ ein Wasserstoffatom oder eine Hydroxylgruppe ist,
R⁵ ein Wasserstoffatom oder eine Methylgruppe ist,
R⁸ 1-Butenyl, 1,3-Butadienyl, n-Butyl, 3-Hydroxy-1-butenyl, Butan-3-on, 1,2-Epoxybutyl oder 1-Propenyl ist,
R⁹ ein Wasserstoffatom oder eine Gruppe ist, ausgewählt aus 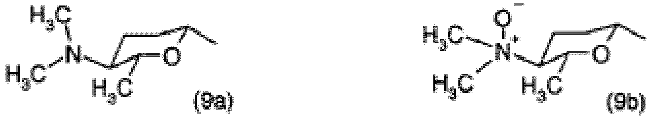 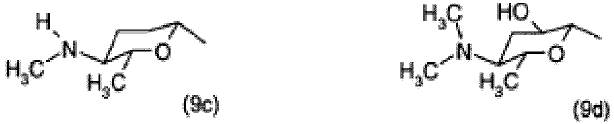 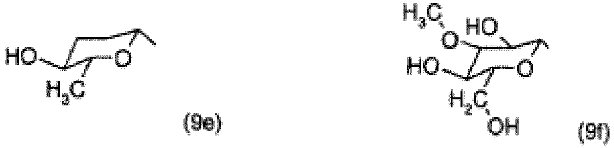 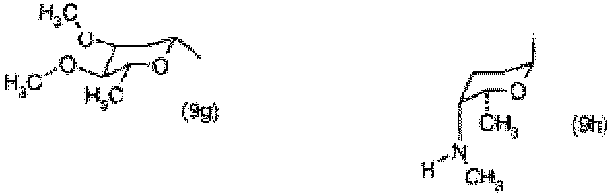 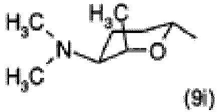 vorausgesetzt, dass:
a) wenn R³ eine Gruppe der Formel (3b) oder (3c) ist, R⁴ und R⁵ = H sind und R⁸ 1-Butenyl ist, dann ist R⁹ weder H noch eine Gruppe der Formel (9a); und
b) wenn R³ eine Gruppe der Formel (3a) ist, dann sind R⁴ und R⁵ = H, R⁸ ist 1-Propenyl und R⁹ ist H.

2. Eine Verbindung gemäß Anspruch 1, worin R⁸ 1-Butenyl, 1,3-Butadienyl, n-Butyl, 3-Hydroxy-1-butenyl oder 1-Propenyl ist.

3. Eine Verbindung gemäß Anspruch 1, worin R³, R⁴, R⁵, R⁸ und R⁹ in einer der folgenden Kombinationen vorliegen:
| Zusammensetzung Nr. | Formel | R³ | R⁴ | R⁵ | R⁸ | R⁹ |
|---|---|---|---|---|---|---|
| 32 | (1A) | (3d) | H | H | 1-Butenyl | (9a) |
| 33 | (1A) | (3f) | H | H | 1-Butenyl | (9a) |
| 34 | (1A) | (3d) | OH | H | 1-Butenyl | (9a) |
| 35 | (1A) | (3d) | H | H | 1-Butenyl | (9c) |
| 36 | (1A) | (3d) | H | CH₃ | 1-Butenyl | (9a) |
| 37 | (1A) | (3e) | H | H | 1-Butenyl | (9a) |
| 38 | (1A) | (3a) | H | H | 1-Propenyl | H |
| 39 | (1A) | (3d) | H | H | 1-Butenyl | H |
| 40 | (1A) | (3e) | OH | H | 1-Butenyl | (9a) |
| 41 | (1A) | (3c) | OH | H | 1-Butenyl | (9a) |
| 42 | (1A) | (3e) | H | H | 1-Butenyl | (9b) |
| 43 | (1A) | (3e) | H | H | 1-Propenyl | (9a) |
| 44 | (1A) | (3e) | H | CH₃ | 1-Butenyl | (9a) |
| 45 | (1A) | (3d) | H | H | 1-Butenyl | (9c) |
| 46 | (1A) | (3d) | H | H | 1-Butenyl | (9d) |
| 47 | (1A) | (3d) | H | H | 3-Hydroxy-1-butenyl | (9a) |
| 48 | (1A) | (3d) | H | H | 3-Hydroxy-1-butenyl | (9c) |
| 49 | (1A) | (3d) | H | H | 1,3-Butadienyl | (9a) |
| 50 | (1A) | (3d) | H | H | 1-Butenyl | (9e) |
| 51 | (1A) | (3d) | H | H | 1-Butenyl | (9f) |
| 52 | (1A) | (3d) | OH | H | 1-Butenyl | (9e) |
| 53 | (1A) | (3d) | OH | H | 1-Butenyl | (9g) |
| 54 | (1A) | (3d) | H | H | 1-Propenyl | (9a) |
| 55 | (1A) | (3d) | H | CH₃ | 1-Butenyl | H |
| 56 | (1A) | (3d) | OH | H | 1-Butenyl | H |
| 57 | (1A) | (3d) | H | H | 3-Hydroxy-1-butenyl | H |
| 58 | (1A) | (3d) | OH | H | 3-Hydroxy-1-butenyl | H |
| 59 | (1A) | (3d) | H | H | 1,3-Butadienyl | H |
| 60 | (1A) | (3d) | H | H | n-Butyl | H |
| 61 | (1A) | (3d) | H | H | 1-Butenyl | (9h) |
| 62 | (1A) | (3d) | H | H | 1-Butenyl | (9i) |
| 63 | (1A) | (3d) | OH | H | 1,3-Butadienyl | (9a) |
| 64 | (1A) | (3d) | OH | CH₃ | 1-Butenyl | (9a) |
| 65 | (1A) | (3d) | OH | H | 1-Propenyl | (9a) |
| 66 | (1A) | (3e) | H | H | 1-Butenyl | (9c) |
| 67 | (1A) | (3e) | H | H | 1-Butenyl | (9d) |
| 68 | (1A) | (3e) | H | H | 3-Hydroxy-1-butenyl | (9a) |
| 69 | (1A) | (3e) | H | H | 3-Hydroxy-1-butenyl | (9c) |
| 70 | (1A) | (3e) | H | H | 1,3-Butadienyl | (9a) |
| 71 | (1A) | (3e) | H | H | 1-Butenyl | (9e) |
| 72 | (1A) | (3e) | H | H | 1-Butenyl | (9f) |
| 73 | (1A) | (3e) | OH | H | 1-Butenyl | (9e) |
| 74 | (1A) | (3e) | OH | H | 1-Butenyl | (9g) |
| 75 | (1A) | (3e) | H | H | 1-Butenyl | H |
| 76 | (1A) | (3e) | H | CH₃ | 1-Butenyl | H |
| 77 | (1A) | (3e) | OH | H | 1-Butenyl | H |
| 78 | (1A) | (3e) | H | H | 3-Hydroxy-1-butenyl | H |
| 79 | (1A) | (3e) | OH | H | 3-Hydroxy-1-butenyl | H |
| 80 | (1A) | (3e) | H | H | 1,3-Butadienyl | H |
| 81 | (1A) | (3e) | H | H | n-Butyl | H |
| 82 | (1A) | (3e) | H | H | 1-Butenyl | (9h) |
| 83 | (1A) | (3e) | H | H | 1-Butenyl | (9i) |
| 84 | (1A) | (3e) | OH | H | 1,3-Butadienyl | (9a) |
| 85 | (1A) | (3e) | OH | CH₃ | 1-Butenyl | (9a) |
| 86 | (1A) | (3e) | OH | H | 1-Propenyl | (9a) |
| 87 | (1A) | (3f) | H | H | 1-Butenyl | (9b) |
| 88 | (1A) | (3f) | H | H | 1-Butenyl | (9c) |
| 89 | (1A) | (3f) | H | H | 1-Butenyl | (9d) |
| 90 | (1A) | (3f) | H | CH₃ | 1-Butenyl | (9a) |
| 91 | (1A) | (3f) | OH | H | 1-Butenyl | (9a) |
| 92 | (1A) | (3f) | H | H | 3-Hydroxy-1-butenyl | (9a) |
| 93 | (1A) | (3f) | H | H | 3-Hydroxy-1-butenyl | (9c) |
| 94 | (1A) | (3f) | H | H | 1,3-Butadienyl | (9a) |
| 95 | (1A) | (3f) | H | H | 1-Butenyl | (9e) |
| 96 | (1A) | (3f) | H | H | 1-Butenyl | (9f) |
| 97 | (1A) | (3f) | OH | H | 1-Butenyl | (9e) |
| 98 | (1A) | (3f) | OH | H | 1-Butenyl | (9g) |
| 99 | (1A) | (3f) | H | H | 1-Propenyl | (9a) |
| 100 | (1A) | (3f) | H | H | 1-Butenyl | H |
| 101 | (1A) | (3f) | H | CH₃ | 1-Butenyl | H |
| 102 | (1A) | (3f) | OH | H | 1-Butenyl | H |
| 103 | (1A) | (3f) | H | H | 3-Hydroxy-1-butenyl | H |
| 104 | (1A) | (3f) | OH | H | 3-Hydroxy-1-butenyl | H |
| 105 | (1A) | (3f) | H | H | 1,3-Butadienyl | H |
| 106 | (1A) | (3f) | H | H | n-Butyl | H |
| 107 | (1A) | (3f) | H | H | 1-Butenyl | (9h) |
| 108 | (1A) | (3f) | H | H | 1-Butenyl | (9i) |
| 109 | (1A) | (3f) | OH | H | 1,3-Butadienyl | (9a) |
| 110 | (1A) | (3f) | OH | CH₃ | 1-Butenyl | (9a) |
| 111 | (1A) | (3f) | OH | H | 1-Propenyl | (9a) |
| 112 | (1A) | (3b) | H | H | 1-Butenyl | (9b) |
| 113 | (1A) | (3b) | H | H | 1-Butenyl | (9c) |
| 114 | (1A) | (3b) | H | H | 1-Butenyl | (9d) |
| 115 | (1A) | (3b) | H | CH₃ | 1-Butenyl | (9a) |
| 116 | (1A) | (3b) | OH | H | 1-Butenyl | (9a) |
| 117 | (1A) | (3b) | H | H | 3-Hydroxy-1-butenyl | (9a) |
| 118 | (1A) | (3b) | H | H | 3-Hydroxy-1-butenyl | (9c) |
| 119 | (1A) | (3b) | H | H | 1,3-Butadienyl | (9a) |
| 120 | (1A) | (3b) | H | H | 1-Butenyl | (9e) |
| 121 | (1A) | (3b) | H | H | 1-Butenyl | (9f) |
| 122 | (1A) | (3b) | OH | H | 1-Butenyl | (9e) |
| 123 | (1A) | (3b) | OH | H | 1-Butenyl | (9g) |
| 124 | (1A) | (3b) | H | H | 1-Propenyl | (9a) |
| 125 | (1A) | (3b) | H | CH₃ | 1-Butenyl | H |
| 126 | (1A) | (3b) | OH | H | 1-Butenyl | H |
| 127 | (1A) | (3b) | H | H | 3-Hydroxy-1-butenyl | H |
| 128 | (1A) | (3b) | OH | H | 3-Hydroxy-1-butenyl | H |
| 129 | (1A) | (3b) | H | H | 1,3-Butadienyl | H |
| 130 | (1A) | (3b) | H | H | n-Butyl | H |
| 131 | (1A) | (3b) | H | H | 1-Butenyl | (9h) |
| 132 | (1A) | (3b) | H | H | 1-Butenyl | (9i) |
| 133 | (1A) | (3b) | OH | H | 1,3-Butadienyl | (9a) |
| 134 | (1A) | (3b) | OH | CH₃ | 1-Butenyl | (9a) |
| 135 | (1A) | (3b) | OH | H | 1-Propenyl | (9a) |
| 136 | (1A) | (3c) | H | H | 1-Butenyl | (9b) |
| 137 | (1A) | (3c) | H | H | 1-Butenyl | (9c) |
| 138 | (1A) | (3c) | H | H | 1-Butenyl | (9d) |
| 139 | (1A) | (3c) | H | CH₃ | 1-Butenyl | (9a) |
| 140 | (1A) | (3c) | OH | H | 1-Butenyl | (9a) |
| 141 | (1A) | (3c) | H | H | 3-Hydroxy-1-butenyl | (9a) |
| 142 | (1A) | (3c) | H | H | 3-Hydroxy-1-butenyl | (9c) |
| 143 | (1A) | (3c) | H | H | 1,3-Butadienyl | (9a) |
| 144 | (1A) | (3c) | H | H | 1-Butenyl | (9e) |
| 145 | (1A) | (3c) | H | H | 1-Butenyl | (9f) |
| 146 | (1A) | (3c) | OH | H | 1-Butenyl | (9e) |
| 147 | (1A) | (3c) | OH | H | 1-Butenyl | (9g) |
| 148 | (1A) | (3c) | H | H | 1-Propenyl | (9a) |
| 149 | (1A) | (3c) | H | CH₃ | 1-Butenyl | H |
| 150 | (1A) | (3c) | OH | H | 1-Butenyl | H |
| 151 | (1A) | (3c) | H | H | 3-Hydroxy-1-butenyl | H |
| 152 | (1A) | (3c) | OH | H | 3-Hydroxy-1-butenyl | H |
| 153 | (1A) | (3c) | H | H | 1,3-Butadienyl | H |
| 154 | (1A) | (3c) | H | H | n-Butyl | H |
| 155 | (1A) | (3c) | H | H | 1-Butenyl | (9h) |
| 156 | (1A) | (3c) | H | H | 1-Butenyl | (9i) |
| 157 | (1A) | (3c) | OH | H | 1,3-Butadienyl | (9a) |
| 158 | (1A) | (3c) | OH | CH₃ | 1-Butenyl | (9a) |
| 159 | (1A) | (3c) | OH | H | 1-Propenyl | (9a) |
| 165 | (1A) | (3c) | H | H | Butan-3-on | (9a) |
| 166 | (1A) | (3c) | H | H | 1,2-Epoxy-butyl | (9a) |
| 167 | (1A) | (3d) | H | H | Butan-3-on | (9a) |
| 168 | (1A) | (3d) | H | H | 1,2-Epoxy-butyl | (9a) |
| 169 | (1A) | (3e) | H | H | Butan-3-on | (9a) |
| 170 | (1A) | (3e) | H | H | 1,2-Epoxy-butyl | (9a) |
| 171 | (1A) | (3e) | H | H | Butan-3-on | (9a) |
| 172 | (1A) | (3e) | H | H | 1,2-Epoxy-butyl | (9a) |
| 173 | (1A) | (3b) | H | H | Butan-3-on | (9a) |
| 174 | (1A) | (3b) | H | H | 1,2-Epoxy-butyl | (9a) |
| 175 | (1A) | (3a) | H | H | Butan-3-on | (9a) |
| 176 | (1A) | (3a) | H | H | 1,2-Epoxy-butyl | (9a) |
| 177 | (1A) | (3a) | OH | H | Butan-3-on | (9a) |
| 178 | (1A) | (3a) | OH | H | 1,2-Epoxy-butyl | (9a) |
| 179 | (1A) | (3a) | H | CH₃ | Butan-3-on | (9a) |
| 180 | (1A) | (3a) | H | CH₃ | 1,2-Epoxy-butyl | (9a) |
| 181 | (1A) | (3a) | OH | CH₃ | Butan-3-on | (9a) |
| 182 | (1A) | (3a) | OH | CH₃ | 1,2-Epoxy-butyl | (9a) |
| 183 | (1A) | (3b) | H | H | Butan-3-on | (9b) |
| 184 | (1A) | (3b) | H | H | Butan-3-on | (9c) |
| 185 | (1A) | (3b) | H | H | Butan-3-on | (9d) |
| 186 | (1A) | (3b) | H | H | Butan-3-on | (9e) |
| 187 | (1A) | (3b) | H | H | Butan-3-on | (9f) |
| 188 | (1A) | (3b) | OH | H | Butan-3-on | (9e) |
| 189 | (1A) | (3b) | OH | H | Butan-3-on | (9g) |
| 190 | (1A) | (3b) | H | CH₃ | Butan-3-on | H |
| 191 | (1A) | (3b) | OH | H | Butan-3-on | H |
| 192 | (1A) | (3b) | H | H | Butan-3-on | (9h) |
| 193 | (1A) | (3b) | H | H | Butan-3-on | (9i) |
| 194 | (1A) | (3b) | H | H | 1,2-Epoxy-butyl | (9b) |
| 195 | (1A) | (3b) | H | H | 1,2-Epoxy-butyl | (9c) |
| 196 | (1A) | (3b) | H | H | 1,2-Epoxy-butyl | (9d) |
| 197 | (1A) | (3b) | H | H | 1,2-Epoxy-butyl | (9e) |
| 199 | (1A) | (3b) | H | H | 1,2-Epoxy-butyl | (9f) |
| 200 | (1A) | (3b) | OH | H | 1,2-Epoxy-butyl | (9e) |
| 201 | (1A) | (3b) | OH | H | 1,2-Epoxy-butyl | (9g) |
| 202 | (1A) | (3b) | H | CH₃ | 1,2-Epoxy-butyl | H |
| 203 | (1A) | (3b) | OH | H | 1,2-Epoxy-butyl | H |
| 204 | (1A) | (3b) | H | H | 1,2-Epoxy-butyl | (9h) |
| 205 | (1A) | (3b) | H | H | 1,2-Epoxy-butyl | (9i) |

4. Eine Verbindung gemäß Anspruch 1 mit der Formel (1A).

5. Eine Verbindung gemäß Anspruch 4, worin R³ R⁴, R⁵, R⁸ und R⁹ in einer der folgenden Kombinationen vorliegen:
| Zusammensetzung Nr. | R³ | R⁴ | R⁵ | R⁸ | R⁹ |
|---|---|---|---|---|---|
| 32 | (3d) | H | H | 1-Butenyl | (9a) |
| 33 | (3f) | H | H | 1-Butenyl | (9a) |
| 34 | (3d) | OH | H | 1-Butenyl | (9a) |
| 35 | (3d) | H | H | 1-Butenyl | (9c) |
| 36 | (3d) | H | CH₃ | 1-Butenyl | (9a) |
| 37 | (3e) | H | H | 1-Butenyl | (9a) |
| 38 | (3a) | H | H | 1-Propenyl | H |
| 39 | (3d) | H | H | 1-Butenyl | H |
| 40 | (3e) | OH | H | 1-Butenyl | (9a) |
| 41 | (3c) | OH | H | 1-Butenyl | (9a) |
| 42 | (3e) | H | H | 1-Butenyl | (9b) |
| 43 | (3e) | H | H | 1-Propenyl | (9a) |
| 44 | (3e) | H | CH₃ | 1-Butenyl | (9a) |
| 45 | (3d) | H | H | 1-Butenyl | (9c) |
| 46 | (3d) | H | H | 1-Butenyl | (9d) |
| 47 | (3d) | H | H | 3-Hydroxy-1-butenyl | (9a) |
| 48 | (3d) | H | H | 3-Hydroxy-1-butenyl | (9c) |
| 49 | (3d) | H | H | 1,3-Butadienyl | (9a) |
| 50 | (3d) | H | H | 1-Butenyl | (9e) |
| 51 | (3d) | H | H | 1-Butenyl | (9f) |
| 52 | (3d) | OH | H | 1-Butenyl | (9e) |
| 53 | (3d) | OH | H | 1-Butenyl | (9g) |
| 54 | (3d) | H | H | 1-Propenyl | (9a) |
| 55 | (3d) | H | CH₃ | 1-Butenyl | H |
| 56 | (3d) | OH | H | 1-Butenyl | H |
| 57 | (3d) | H | H | 3-Hydroxy-1-butenyl | H |
| 58 | (3d) | OH | H | 3-Hydroxy-1-butenyl | H |
| 59 | (3d) | H | H | 1,3-Butadienyl | H |
| 60 | (3d) | H | H | n-Butyl | H |
| 61 | (3d) | H | H | 1-Butenyl | (9h) |
| 62 | (3d) | H | H | 1-Butenyl | (9i) |
| 63 | (3d) | OH | H | 1,3-Butadienyl | (9a) |
| 64 | (3d) | OH | CH₃ | 1-Butenyl | (9a) |
| 65 | (3d) | OH | H | 1-Propenyl | (9a) |
| 66 | (3e) | H | H | 1-Butenyl | (9c) |
| 67 | (3e) | H | H | 1-Butenyl | (9d) |
| 68 | (3e) | H | H | 3-Hydroxy-1-butenyl | (9a) |
| 69 | (3e) | H | H | 3-Hydroxy-1-butenyl | (9c) |
| 70 | (3e) | H | H | 1,3-Butadienyl | (9a) |
| 71 | (3e) | H | H | 1-Butenyl | (9e) |
| 72 | (3e) | H | H | 1-Butenyl | (9f) |
| 73 | (3e) | OH | H | 1-Butenyl | (9e) |
| 74 | (3e) | OH | H | 1-Butenyl | (9g) |
| 75 | (3e) | H | H | 1-Butenyl | H |
| 76 | (3e) | H | CH₃ | 1-Butenyl | H |
| 77 | (3e) | OH | H | 1-Butenyl | H |
| 78 | (3e) | H | H | 3-Hydroxy-1-butenyl | H |
| 79 | (3e) | OH | H | 3-Hydroxy-1-butenyl | H |
| 80 | (3e) | H | H | 1,3-Butadienyl | H |
| 81 | (3e) | H | H | n-Butyl | H |
| 82 | (3e) | H | H | 1-Butenyl | (9h) |
| 83 | (3e) | H | H | 1-Butenyl | (9i) |
| 84 | (3e) | OH | H | 1,3-Butadienyl | (9a) |
| 85 | (3e) | OH | CH₃ | 1-Butenyl | (9a) |
| 86 | (3e) | OH | H | 1-Propenyl | (9a) |
| 87 | (3f) | H | H | 1-Butenyl | (9b) |
| 88 | (3f) | H | H | 1-Butenyl | (9c) |
| 89 | (3f) | H | H | 1-Butenyl | (9d) |
| 90 | (3f) | H | CH₃ | 1-Butenyl | (9a) |
| 91 | (3f) | OH | H | 1-Butenyl | (9a) |
| 92 | (3f) | H | H | 3-Hydroxy-1-butenyl | (9a) |
| 93 | (3f) | H | H | 3-Hydroxy-1-butenyl | (9c) |
| 94 | (3f) | H | H | 1,3-Butadienyl | (9a) |
| 95 | (3f) | H | H | 1-Butenyl | (9e) |
| 96 | (3f) | H | H | 1-Butenyl | (9f) |
| 97 | (3f) | OH | H | 1-Butenyl | (9e) |
| 98 | (3f) | OH | H | 1-Butenyl | (9g) |
| 99 | (3f) | H | H | 1-Propenyl | (9a) |
| 100 | (3f) | H | H | 1-Butenyl | H |
| 101 | (3f) | H | CH₃ | 1-Butenyl | H |
| 102 | (3f) | OH | H | 1-Butenyl | H |
| 103 | (3f) | H | H | 3-Hydroxy-1-butenyl | H |
| 104 | (3f) | OH | H | 3-Hydroxy-1-butenyl | H |
| 105 | (3f) | H | H | 1,3-Butadienyl | H |
| 106 | (3f) | H | H | n-Butyl | H |
| 107 | (3f) | H | H | 1-Butenyl | (9h) |
| 108 | (3f) | H | H | 1-Butenyl | (9i) |
| 109 | (3f) | OH | H | 1,3-Butadienyl | (9a) |
| 110 | (3f) | OH | CH₃ | 1-Butenyl | (9a) |
| 111 | (3f) | OH | H | 1-Propenyl | (9a) |
| 112 | (3b) | H | H | 1-Butenyl | (9b) |
| 113 | (3b) | H | H | 1-Butenyl | (9c) |
| 114 | (3b) | H | H | 1-Butenyl | (9d) |
| 115 | (3b) | H | CH₃ | 1-Butenyl | (9a) |
| 116 | (3b) | OH | H | 1-Butenyl | (9a) |
| 117 | (3b) | H | H | 3-Hydroxy-1-butenyl | (9a) |
| 118 | (3b) | H | H | 3-Hydroxy-1-butenyl | (9c) |
| 119 | (3b) | H | H | 1,3-Butadienyl | (9a) |
| 120 | (3b) | H | H | 1-Butenyl | (9e) |
| 121 | (3b) | H | H | 1-Butenyl | (9f) |
| 122 | (3b) | OH | H | 1-Butenyl | (9e) |
| 123 | (3b) | OH | H | 1-Butenyl | (9g) |
| 124 | (3b) | H | H | 1-Propenyl | (9a) |
| 125 | (3b) | H | CH₃ | 1-Butenyl | H |
| 126 | (3b) | OH | H | 1-Butenyl | H |
| 127 | (3b) | H | H | 3-Hydroxy-1-butenyl | H |
| 128 | (3b) | OH | H | 3-Hydroxy-1-butenyl | H |
| 129 | (3b) | H | H | 1,3-Butadienyl | H |
| 130 | (3b) | H | H | n-Butyl | H |
| 131 | (3b) | H | H | 1-Butenyl | (9h) |
| 132 | (3b) | H | H | 1-Butenyl | (9i) |
| 133 | (3b) | OH | H | 1,3-Butadienyl | (9a) |
| 134 | (3b) | OH | CH₃ | 1-Butenyl | (9a) |
| 135 | (3b) | OH | H | 1-Propenyl | (9a) |
| 136 | (3c) | H | H | 1-Butenyl | (9b) |
| 137 | (3c) | H | H | 1-Butenyl | (9c) |
| 138 | (3c) | H | H | 1-Butenyl | (9d) |
| 139 | (3c) | H | CH₃ | 1-Butenyl | (9a) |
| 140 | (3c) | OH | H | 1-Butenyl | (9a) |
| 141 | (3c) | H | H | 3-Hydroxy-1-butenyl | (9a) |
| 142 | (3c) | H | H | 3-Hydroxy-1-butenyl | (9c) |
| 143 | (3c) | H | H | 1,3-Butadienyl | (9a) |
| 144 | (3c) | H | H | 1-Butenyl | (9e) |
| 145 | (3c) | H | H | 1-Butenyl | (9f) |
| 146 | (3c) | OH | H | 1-Butenyl | (9e) |
| 147 | (3c) | OH | H | 1-Butenyl | (9g) |
| 148 | (3c) | H | H | 1-Propenyl | (9a) |
| 149 | (3c) | H | CH₃ | 1-Butenyl | H |
| 150 | (3c) | OH | H | 1-Butenyl | H |
| 151 | (3c) | H | H | 3-Hydroxy-1-butenyl | H |
| 152 | (3c) | OH | H | 3-Hydroxy-1-butenyl | H |
| 153 | (3c) | H | H | 1,3-Butadienyl | H |
| 154 | (3c) | H | H | n-Butyl | H |
| 155 | (3c) | H | H | 1-Butenyl | (9h) |
| 156 | (3c) | H | H | 1-Butenyl | (9i) |
| 157 | (3c) | OH | H | 1,3-Butadienyl | (9a) |
| 158 | (3c) | OH | CH₃ | 1-Butenyl | (9a) |
| 159 | (3c) | OH | H | 1-Propenyl | (9a) |

6. Eine Verbindung der Formel (10) :

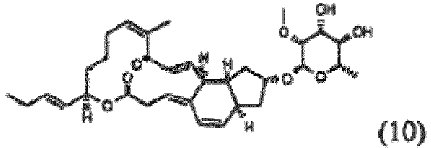

7. Eine Verbindung der Formel (11):

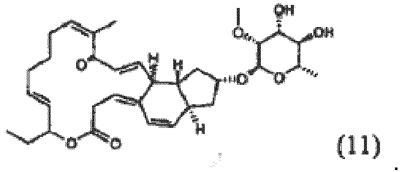

8. Eine Verbindung gemäß Anspruch 1, worin R⁹ etwas anderes als H ist.

9. Eine Verbindung gemäß Anspruch 3, worin R⁹ etwas anderes als H ist.

10. Eine insektizide oder akarizide (Milben bekämpfende) Zusammensetzung, die eine ein Insekt, eine Milbe oder einen Holzbock inaktivierende Menge einer Verbindung gemäß Anspruch 8 im Kombination mit einem phytologisch oder physiologisch annehmbaren Träger enthält.

11. Eine Verbindung gemäß Anspruch 8 für die Verwendung als ein Insektizid oder Akarizid.

12. Eine Verbindung gemäß Anspruch 8 für die Verwendung zum Schutz eines Ortes vor Befall durch Insekten, Milben oder Holzböcke.

13. Eine Verbindung gemäß Anspruch 8 für die Verwendung zum Unter-Kontrolle-Halten einer Population von Parasiten, die ein Wirtstier befallen.

14. Eine Formulierung zur Verwendung zum Unter-Kontrolle-Halten eines Läusebefalls bei einem Menschen, enthaltend als einen aktiven Bestandteil eine Verbindung gemäß Anspruch 8, oder ein physiologisch annehmbares Derivat oder Salz davon und einen physiologisch annehmbaren Träger.

15. Eine Verbindung gemäß Anspruch 8 für die Verwendung zum Unter-Kontrolle-Halten eines Läusebefalls bei einem Menschen.

16. Ein Verfahren zur Herstellung einer Verbindung gemäß Anspruch 1, welches das Kultivieren eines Stammes, ausgewählt aus
NRRL 30424,
NRRL 30423,
NRRL 30422,
NRRL 30438,
NRRL 30421 und
NRRL 30437
oder eines Stammes, der davon abgeleitet ist, der eine Verbindung gemäß Anspruch 1 herstellt, in einem geeigneten Kulturmedium unter aeroben Fermentationsbedingungen in Flüssig-Schüttelkultur, bis eine ausbeutbare Menge einer Verbindung gemäß Anspruch 1 hergestellt ist und Gewinnen einer Verbindung gemäß Anspruch 1 aus dem Kulturmedium, umfasst.

17. Eine biologisch reine Kultur von NRRL 30424 oder eines Stammes, der davon abgeleitet ist, die eine Verbindung gemäß Anspruch 1 herstellt.

18. Eine biologisch reine Kultur von NRRL 30423 oder eines Stammes, der davon abgeleitet ist, die eine Verbindung gemäß Anspruch 1 herstellt.

19. Eine biologisch reine Kultur von NRRL 30422 oder eines Stammes, der davon abgeleitet ist, die eine Verbindung gemäß Anspruch 1 herstellt.

20. Eine biologisch reine Kultur von NRRL 30438 oder eines Stammes, der davon abgeleitet ist, die eine Verbindung gemäß Anspruch 1 herstellt.

21. Eine biologisch reine Kultur von NRRL 30421 oder eines Stammes, der davon abgeleitet ist, die eine Verbindung gemäß Anspruch 1 herstellt.

22. Eine biologisch reine Kultur von NRRL 30437 oder eines Stammes, der davon abgeleitet ist, die eine Verbindung gemäß Anspruch 1 herstellt.

## Revendications

1. Composé de formule (1A) ou (2A) 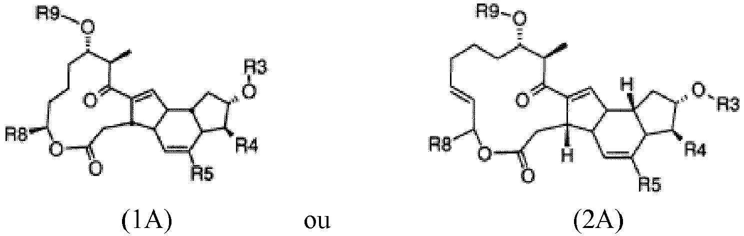 formules dans lesquelles :
R³ est un groupe choisi parmi 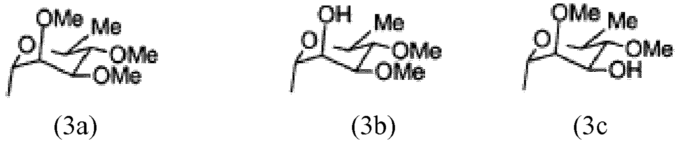 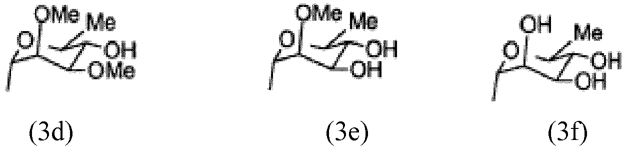
R⁴ est un atome d'hydrogène ou un groupe hydroxy,
R⁵ est un atome d'hydrogène ou le groupe méthyle,
R⁸ est le groupe 1-butényle, 1,3-butadiényle, n-butyle, 3-hydroxy-1-butényle, butan-3-one, 1,2-époxy-butyle ou 1-propényle,
R⁹ est un atome d'hydrogène ou un groupe choisi parmi 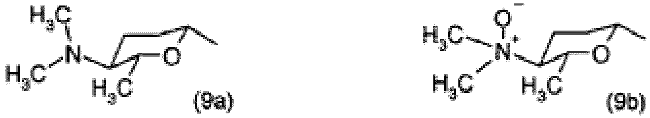 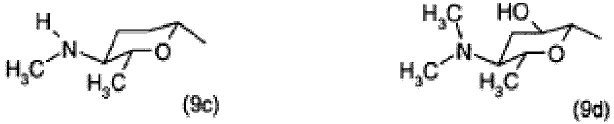 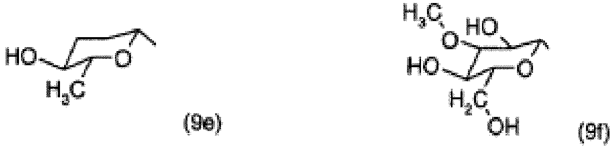 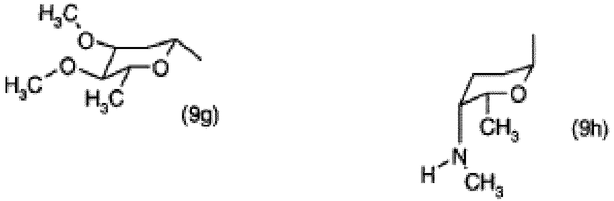 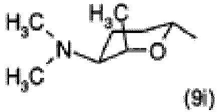
étant entendu que :
a) si R³ est un groupe de formule (3b) ou (3c), R⁴ et R⁵ représentent H, et R⁸ est le groupe 1-butényle, alors R⁹ n'est ni H ni un groupe de formule (9a) ; et
b) si R³ est un groupe de formule (3a), alors R⁴ et R⁵ représentent H, R⁸ est le groupe 1-propényle, et R⁹ est H.

2. Composé selon la revendication 1, dans lequel R⁸ est le groupe 1-butényle, 1,3-butadiényle, n-butyle, 3-hydroxy-1-butényle ou 1-propényle.

3. Composé selon la revendication 1, dans lequel R³, R⁴, R⁵, R⁸ et R⁹ sont présents en l'une des combinaisons suivantes :
| Composé n° | Formule | R³ | R⁴ | R⁵ | R⁸ | R⁹ |
|---|---|---|---|---|---|---|
| 32 | (1A) | (3d) | H | H | 1-butényle | (9a) |
| 33 | (1A) | (3f) | H | H | 1-butényle | (9a) |
| 34 | (1A) | (3d) | OH | H | 1-butényle | (9a) |
| 35 | (1A) | (3d) | H | CH₃ | 1-butényle | (9c) |
| 36 | (1A) | (3d) | H | CH₃ | 1-butényle | (9a) |
| 37 | (1A) | (3e) | H | H | 1-butényle | (9a) |
| 38 | (1A) | (3a) | H | H | 1-propényle | H |
| 39 | (1A) | (3d) | H | H | 1-butényle | H |
| 40 | (1A) | (3e) | OH | H | 1-butényle | (9a) |
| 41 | (1A) | (3c) | OH | H | 1-butényle | (9a) |
| 42 | (1A) | (3e) | H | H | 1-butényle | (9b) |
| 43 | (1A) | (3e) | H | H | 1-propényle | (9a) |
| 44 | (1A) | (3e) | H | CH₃ | 1-butényle | (9a) |
| 45 | (1A) | (3d) | H | H | 1-butényle | (9c) |
| 46 | (1A) | (3d) | H | H | 1-butényle | (9d) |
| 47 | (1A) | (3d) | H | H | 3-hydroxy-1-butényle | (9a) |
| 48 | (1A) | (3d) | H | H | 3-hydroxy-1-butényle | (9c) |
| 49 | (1A) | (3d) | H | H | 1,3-butadiényle | (9a) |
| 50 | (1A) | (3d) | H | H | 1-butényle | (9e) |
| 51 | (1A) | (3d) | H | H | 1-butényle | (9f) |
| 52 | (1A) | (3d) | OH | H | 1-butényle | (9e) |
| 53 | (1A) | (3d) | OH | H | 1-butényle | (9g) |
| 54 | (1A) | (3d) | H | H | 1-propényle | (9a) |
| 55 | (1A) | (3d) | H | CH₃ | 1-butényle | H |
| 56 | (1A) | (3d) | OH | H | 1-butényle | H |
| 57 | (1A) | (3d) | H | H | 3-hydroxy-1-butényle | H |
| 58 | (1A) | (3d) | OH | H | 3-hydroxy-1-butényle | H |
| 59 | (1A) | (3d) | H | H | 1,3-butadiényle | H |
| 60 | (1A) | (3d) | H | H | n-butyle | H |
| 61 | (1A) | (3d) | H | H | 1-butényle | (9h) |
| 62 | (1A) | (3d) | H | H | 1-butényle | (9i) |
| 63 | (1A) | (3d) | OH | H | 1,3-butadiényle | (9a) |
| 64 | (1A) | (3d) | OH | CH₃ | 1-butényle | (9a) |
| 65 | (1A) | (3d) | OH | H | 1-propényle | (9a) |
| 66 | (1A) | (3e) | H | H | 1-butényle | (9c) |
| 67 | (1A) | (3e) | H | H | 1-butényle | (9d) |
| 68 | (1A) | (3e) | H | H | 3-hydroxy-1-butényle | (9a) |
| 69 | (1A) | (3e) | H | H | 3-hydroxy-1-butényle | (9c) |
| 70 | (1A) | (3e) | H | H | 1,3-butadiényle | (9a) |
| 71 | (1A) | (3e) | H | H | 1-butényle | (9e) |
| 72 | (1A) | (3e) | H | H | 1-butényle | (9f) |
| 73 | (1A) | (3e) | OH | H | 1-butényle | (9e) |
| 74 | (1A) | (3e) | OH | H | 1-butényle | (9g) |
| 75 | (1A) | (3e) | H | H | 1-butényle | H |
| 76 | (1A) | (3e) | H | CH₃ | 1-butényle | H |
| 77 | (1A) | (3e) | OH | H | 1-butényle | H |
| 78 | (1A) | (3e) | H | H | 3-hydroxy-1-butényle | H |
| 79 | (1A) | (3e) | OH | H | 3-hydroxy-1-butényle | H |
| 80 | (1A) | (3e) | H | H | 1,3-butadiényle | H |
| 81 | (1A) | (3e) | H | H | n-butyle | H |
| 82 | (1A) | (3e) | H | H | 1-butényle | (9h) |
| 83 | (1A) | (3e) | H | H | 1-butényle | (9i) |
| 84 | (1A) | (3e) | OH | H | 1,3-butadiényle | (9a) |
| 85 | (1A) | (3e) | OH | CH₃ | 1-butényle | (9a) |
| 86 | (1A) | (3e) | OH | H | 1-propényle | (9a) |
| 87 | (1A) | (3f) | H | H | 1-butényle | (9b) |
| 88 | (1A) | (3f) | H | H | 1-butényle | (9c) |
| 89 | (1A) | (3f) | H | H | 1-butényle | (9d) |
| 90 | (1A) | (3f) | H | CH₃ | 1-butényle | (9a) |
| 91 | (1A) | (3f) | OH | H | 1-butényle | (9a) |
| 92 | (1A) | (3f) | H | H | 3-hydroxy-1-butényle | (9a) |
| 93 | (1A) | (3f) | H | H | 3-hydroxy-1-butényle | (9c) |
| 94 | (1A) | (3f) | H | H | 1,3-butadiényle | (9a) |
| 95 | (1A) | (3f) | H | H | 1-butényle | (9e) |
| 96 | (1A) | (3f) | H | H | 1-butényle | (9f) |
| 97 | (1A) | (3f) | OH | H | 1-butényle | (9e) |
| 98 | (1A) | (3f) | OH | H | 1-butényle | (9g) |
| 99 | (1A) | (3f) | H | H | 1-propényle | (9a) |
| 100 | (1A) | (3f) | H | H | 1-butényle | H |
| 101 | (1A) | (3f) | H | CH₃ | 1-butényle | H |
| 102 | (1A) | (3f) | OH | H | 1-butényle | H |
| 103 | (1A) | (3f) | H | H | 3-hydroxy-1-butényle | H |
| 104 | (1A) | (3f) | OH | H | 3-hydroxy-1-butényle | H |
| 105 | (1A) | (3f) | H | H | 1,3-butadiényle | H |
| 106 | (1A) | (3f) | H | H | n-butyle | H |
| 107 | (1A) | (3f) | H | H | 1-butényle | (9h) |
| 108 | (1A) | (3f) | H | H | 1-butényle | (9i) |
| 109 | (1A) | (3f) | OH | H | 1,3-butadiényle | (9a) |
| 110 | (1A) | (3f) | OH | CH₃ | 1-butényle | (9a) |
| 111 | (1A) | (3f) | OH | H | 1-propényle | (9a) |
| 112 | (1A) | (3b) | H | H | 1-butényle | (9b) |
| 113 | (1A) | (3b) | H | H | 1-butényle | (9c) |
| 114 | (1A) | (3b) | H | H | 1-butényle | (9d) |
| 115 | (1A) | (3b) | H | CH₃ | 1-butényle | (9a) |
| 116 | (1A) | (3b) | OH | H | 1-butényle | (9a) |
| 117 | (1A) | (3b) | H | H | 3-hydroxy-1-butényle | (9a) |
| 118 | (1A) | (3b) | H | H | 3-hydroxy-1-butényle | (9c) |
| 119 | (1A) | (3b) | H | H | 1,3-butadiényle | (9a) |
| 120 | (1A) | (3b) | H | H | 1-butényle | (9e) |
| 121 | (1A) | (3b) | H | H | 1-butényle | (9f) |
| 122 | (1A) | (3b) | OH | H | 1-butényle | (9e) |
| 123 | (1A) | (3b) | OH | H | 1-butényle | (9g) |
| 124 | (1A) | (3b) | H | H | 1-propényle | (9a) |
| 125 | (1A) | (3b) | H | CH₃ | 1-butényle | H |
| 126 | (1A) | (3b) | OH | H | 1-butényle | H |
| 127 | (1A) | (3b) | H | H | 3-hydroxy-1-butényle | H |
| 128 | (1A) | (3b) | OH | H | 3-hydroxy-1-butényle | H |
| 129 | (1A) | (3b) | H | H | 1,3-butadiényle | H |
| 130 | (1A) | (3b) | H | H | n-butyle | H |
| 131 | (1A) | (3b) | H | H | 1-butényle | (9h) |
| 132 | (1A) | (3b) | H | H | 1-butényle | (9i) |
| 133 | (1A) | (3b) | OH | H | 1,3-butadiényle | (9a) |
| 134 | (1A) | (3b) | OH | CH₃ | 1-butényle | (9a) |
| 135 | (1A) | (3b) | OH | H | 1-propényle | (9a) |
| 136 | (1A) | (3c) | H | H | 1-butényle | (9b) |
| 137 | (1A) | (3c) | H | H | 1-butényle | (9c) |
| 138 | (1A) | (3c) | H | H | 1-butényle | (9d) |
| 139 | (1A) | (3c) | H | CH₃ | 1-butényle | (9a) |
| 140 | (1A) | (3c) | OH | H | 1-butényle | (9a) |
| 141 | (1A) | (3c) | H | H | 3-hydroxy-1-butényle | (9a) |
| 142 | (1A) | (3c) | H | H | 3-hydroxy-1-butényle | (9c) |
| 143 | (1A) | (3c) | H | H | 1,3-butadiényle | (9a) |
| 144 | (1A) | (3c) | H | H | 1-butényle | (9e) |
| 145 | (1A) | (3c) | H | H | 1-butényle | (9f) |
| 146 | (1A) | (3c) | OH | H | 1-butényle | (9e) |
| 147 | (1A) | (3c) | OH | H | 1-butényle | (9g) |
| 148 | (1A) | (3c) | H | H | 1-propényle | H |
| 149 | (1A) | (3c) | H | CH₃ | 1-butényle | H |
| 150 | (1A) | (3c) | OH | H | 1-butényle | H |
| 151 | (1A) | (3c) | H | H | 3-hydroxy-1-butényle | H |
| 152 | (1A) | (3c) | OH | H | 3-hydroxy-1-butényle | H |
| 153 | (1A) | (3c) | H | H | 1,3-butadiényle | H |
| 154 | (1A) | (3c) | H | H | n-butyle | H |
| 155 | (1A) | (3c) | H | H | 1-butényle | (9h) |
| 156 | (1A) | (3c) | H | H | 1-butényle | (9i) |
| 157 | (1A) | (3c) | OH | H | 1,3-butadiényle | (9a) |
| 158 | (1A) | (3c) | OH | CH₃ | 1-butényle | (9a) |
| 159 | (1A) | (3c) | OH | H | 1-propényle | (9a) |
| 165 | (1A) | (3c) | H | H | butan-3-one | (9a) |
| 166 | (1A) | (3c) | H | H | 1,2-époxy-butyle | (9a) |
| 167 | (1A) | (3d) | H | H | butan-3-one | (9a) |
| 168 | (1A) | (3d) | H | H | 1,2-époxy-butyle | (9a) |
| 169 | (1A) | (3e) | H | H | butan-3-one | (9a) |
| 170 | (1A) | (3e) | H | H | 1,2-époxy-butyle | (9a) |
| 171 | (1A) | (3e) | H | H | butan-3-one | (9a) |
| 172 | (1A) | (3e) | H | H | 1,2-époxy-butyle | (9a) |
| 173 | (1A) | (3b) | H | H | butan-3-one | (9a) |
| 174 | (1A) | (3b) | H | H | 1,2-époxy-butyle | (9a) |
| 175 | (1A) | (3a) | H | H | butan-3-one | (9a) |
| 176 | (1A) | (3a) | H | H | 1,2-époxy-butyle | (9a) |
| 177 | (1A) | (3a) | OH | H | butan-3-one | (9a) |
| 178 | (1A) | (3a) | OH | H | 1,2-époxy-butyle | (9a) |
| 179 | (1A) | (3a) | H | CH₃ | butan-3-one | (9a) |
| 180 | (1A) | (3a) | H | CH₃ | 1,2-époxy-butyle | (9a) |
| 181 | (1A) | (3a) | OH | CH₃ | butan-3-one | (9a) |
| 182 | (1A) | (3a) | OH | CH₃ | 1,2-époxy-butyle | (9a) |
| 183 | (1A) | (3b) | H | H | butan-3-one | (9b) |
| 184 | (1A) | (3b) | H | H | butan-3-one | (9c) |
| 185 | (1A) | (3b) | H | H | butan-3-one | (9d) |
| 186 | (1A) | (3b) | H | H | butan-3-one | (9e) |
| 187 | (1A) | (3b) | H | H | butan-3-one | (9f) |
| 188 | (1A) | (3b) | OH | H | butan-3-one | (9e) |
| 189 | (1A) | (3b) | OH | H | butan-3-one | (9g) |
| 190 | (1A) | (3b) | H | CH₃ | butan-3-one | H |
| 191 | (1A) | (3b) | OH | H | butan-3-one | H |
| 192 | (1A) | (3b) | H | H | butan-3-one | (9h) |
| 193 | (1A) | (3b) | H | H | butan-3-one | (9i) |
| 194 | (1A) | (3b) | H | H | 1,2-époxy-butyle | (9b) |
| 195 | (1A) | (3b) | H | H | 1,2-époxy-butyle | (9c) |
| 196 | (1A) | (3b) | H | H | 1,2-époxy-butyle | (9d) |
| 197 | (1A) | (3b) | H | H | 1,2-époxy-butyle | (9e) |
| 199 | (1A) | (3b) | H | H | 1,2-époxy-butyle | (9f) |
| 200 | (1A) | (3b) | OH | H | 1,2-époxy-butyle | (9e) |
| 201 | (1A) | (3b) | OH | H | 1,2-époxy-butyle | (9g) |
| 202 | (1A) | (3b) | H | CH₃ | 1,2-époxy-butyle | H |
| 203 | (1A) | (3b) | OH | H | 1,2-époxy-butyle | H |
| 204 | (1A) | (3b) | H | H | 1,2-époxy-butyle | (9h) |
| 205 | (1A) | (3b) | H | H | 1,2-époxy-butyle | (9i) |

4. Composé selon la revendication 1, de formule (1A).

5. Composé selon la revendication 4, dans lequel R³, R⁴, R⁵, R⁸ et R⁹ sont présents en l'une des combinaisons suivantes :
| Composé n° | R³ | R⁴ | R⁵ | R⁸ | R⁹ |
|---|---|---|---|---|---|
| 32 | (3d) | H | H | 1-butényle | (9a) |
| 33 | (3f) | H | H | 1-butényle | (9a) |
| 34 | (3d) | OH | H | 1-butényle | (9a) |
| 35 | (3d) | H | H | 1-butényle | (9c) |
| 36 | (3d) | H | CH₃ | 1-butényle | (9a) |
| 37 | (3e) | H | H | 1-butényle | (9a) |
| 38 | (3a) | H | H | 1-propényle | H |
| 39 | (3d) | H | H | 1-butényle | H |
| 40 | (3e) | OH | H | 1-butényle | (9a) |
| 41 | (3c) | OH | H | 1-butényle | (9a) |
| 42 | (3e) | H | H | 1-butényle | (9b) |
| 43 | (3e) | H | H | 1-propényle | (9a) |
| 44 | (3e) | H | CH₃ | 1-butényle | (9a) |
| 45 | (3d) | H | H | 1-butényle | (9c) |
| 46 | (3d) | H | H | 1-butényle | (9d) |
| 47 | (3d) | H | H | 3-hydroxy-1-butényle | (9a) |
| 48 | (3d) | H | H | 3-hydroxy-1-butényle | (9c) |
| 49 | (3d) | H | H | 1,3-butadiényle | (9a) |
| 50 | (3d) | H | H | 1-butényle | (9e) |
| 51 | (3d) | H | H | 1-butényle | (9f) |
| 52 | (3d) | OH | H | 1-butényle | (9e) |
| 53 | (3d) | OH | H | 1-butényle | (9g) |
| 54 | (3d) | H | H | 1-propényle | (9a) |
| 55 | (3d) | H | CH₃ | 1-butényle | H |
| 56 | (3d) | OH | H | 1-butényle | H |
| 57 | (3d) | H | H | 3-hydroxy-1-butényle | H |
| 58 | (3d) | OH | H | 3-hydroxy-1-butényle | H |
| 59 | (3d) | H | H | 1,3-butadiényle | H |
| 60 | (3d) | H | H | n-butyle | H |
| 61 | (3d) | H | H | 1-butényle | (9h) |
| 62 | (3d) | H | H | 1-butényle | (9i) |
| 63 | (3d) | OH | H | 1,3-butadiényle | (9a) |
| 64 | (3d) | OH | CH₃ | 1-butényle | (9a) |
| 65 | (3d) | OH | H | 1-propényle | (9a) |
| 66 | (3e) | H | H | 1-butényle | (9c) |
| 67 | (3e) | H | H | 1-butényle | (9d) |
| 68 | (3e) | H | H | 3-hydroxy-1-butényle | (9a) |
| 69 | (3e) | H | H | 3-hydroxy-1-butényle | (9c) |
| 70 | (3e) | H | H | 1,3-butadiényle | (9a) |
| 71 | (3e) | H | H | 1-butényle | (9e) |
| 72 | (3e) | H | H | 1-butényle | (9f) |
| 73 | (3e) | OH | H | 1-butényle | (9e) |
| 74 | (3e) | OH | H | 1-butényle | (9g) |
| 75 | (3e) | H | H | 1-butényle | H |
| 76 | (3e) | H | CH₃ | 1-butényle | H |
| 77 | (3e) | OH | H | 1-butényle | H |
| 78 | (3e) | H | H | 3-hydroxy-1-butényle | H |
| 79 | (3e) | OH | H | 3-hydroxy-1-butényle | H |
| 80 | (3e) | H | H | 1,3-butadiényle | H |
| 81 | (3e) | H | H | n-butyle | H |
| 82 | (3e) | H | H | 1-butényle | (9h) |
| 83 | (3e) | H | H | 1-butényle | (9i) |
| 84 | (3e) | OH | H | 1,3-butadiényle | (9a) |
| 85 | (3e) | OH | CH₃ | 1-butényle | (9a) |
| 86 | (3e) | OH | H | 1-propényle | (9a) |
| 87 | (3f) | H | H | 1-butényle | (9b) |
| 88 | (3f) | H | H | 1-butényle | (9c) |
| 89 | (3f) | H | H | 1-butényle | (9d) |
| 90 | (3f) | H | CH₃ | 1-butényle | (9a) |
| 91 | (3f) | OH | H | 1-butényle | (9a) |
| 92 | (3f) | H | H | 3-hydroxy-1-butényle | (9a) |
| 93 | (3f) | H | H | 3-hydroxy-1-butényle | (9c) |
| 94 | (3f) | H | H | 1,3-butadiényle | (9a) |
| 95 | (3f) | H | H | 1-butényle | (9e) |
| 96 | (3f) | H | H | 1-butényle | (9f) |
| 97 | (3f) | OH | H | 1-butényle | (9e) |
| 98 | (3f) | OH | H | 1-butényle | (9g) |
| 99 | (3f) | H | H | 1-propényle | (9a) |
| 100 | (3f) | H | H | 1-butényle | H |
| 101 | (3f) | H | CH₃ | 1-butényle | H |
| 102 | (3f) | OH | H | 1-butényle | H |
| 103 | (3f) | H | H | 3-hydroxy-1-butényle | H |
| 104 | (3f) | OH | H | 3-hydroxy-1-butényle | H |
| 105 | (3f) | H | H | 1,3-butadiényle | H |
| 106 | (3f) | H | H | n-butyle | H |
| 107 | (3f) | H | H | 1-butényle | (9h) |
| 108 | (3f) | H | H | 1-butényle | (9i) |
| 109 | (3f) | OH | H | 1,3-butadiényle | (9a) |
| 110 | (3f) | OH | CH₃ | 1-butényle | (9a) |
| 111 | (3f) | OH | H | 1-propényle | (9a) |
| 112 | (3b) | H | H | 1-butényle | (9b) |
| 113 | (3b) | H | H | 1-butényle | (9c) |
| 114 | (3b) | H | H | 1-butényle | (9d) |
| 115 | (3b) | H | CH₃ | 1-butényle | (9a) |
| 116 | (3b) | OH | H | 1-butényle | (9a) |
| 117 | (3b) | H | H | 3-hydroxy-1-butényle | (9a) |
| 118 | (3b) | H | H | 3-hydroxy-1-butényle | (9c) |
| 119 | (3b) | H | H | 1,3-butadiényle | (9a) |
| 120 | (3b) | H | H | 1-butényle | (9e) |
| 121 | (3b) | H | H | 1-butényle | (9f) |
| 122 | (3b) | OH | H | 1-butényle | (9e) |
| 123 | (3b) | OH | H | 1-butényle | (9g) |
| 124 | (3b) | H | H | 1-propényle | (9a) |
| 125 | (3b) | H | CH₃ | 1-butényle | H |
| 126 | (3b) | OH | H | 1-butényle | H |
| 127 | (3b) | H | H | 3-hydroxy-1-butényle | H |
| 128 | (3b) | OH | H | 3-hydroxy-1-butényle | H |
| 129 | (3b) | H | H | 1,3-butadiényle | H |
| 130 | (3b) | H | H | n-butyle | H |
| 131 | (3b) | H | H | 1-butényle | (9h) |
| 132 | (3b) | H | H | 1-butényle | (9i) |
| 133 | (3b) | OH | H | 1,3-butadiényle | (9a) |
| 134 | (3b) | OH | CH₃ | 1-butényle | (9a) |
| 135 | (3b) | OH | H | 1-propényle | (9a) |
| 136 | (3c) | H | H | 1-butényle | (9b) |
| 137 | (3c) | H | H | 1-butényle | (9c) |
| 138 | (3c) | H | H | 1-butényle | (9d) |
| 139 | (3c) | H | CH₃ | 1-butényle | (9a) |
| 140 | (3c) | OH | H | 1-butényle | (9a) |
| 141 | (3c) | H | H | 3-hydroxy-1-butényle | (9a) |
| 142 | (3c) | H | H | 3-hydroxy-1-butényle | (9c) |
| 143 | (3c) | H | H | 1,3-butadiényle | (9a) |
| 144 | (3c) | H | H | 1-butényle | (9e) |
| 145 | (3c) | H | H | 1-butényle | (9f) |
| 146 | (3c) | OH | H | 1-butényle | (9e) |
| 147 | (3c) | OH | H | 1-butényle | (9g) |
| 148 | (3c) | H | H | 1-propényle | (9a) |
| 149 | (3c) | H | CH₃ | 1-butényle | H |
| 150 | (3c) | OH | H | 1-butényle | H |
| 151 | (3c) | H | H | 3-hydroxy-1-butényle | H |
| 152 | (3c) | OH | H | 3-hydroxy-1-butényle | H |
| 153 | (3c) | H | H | 1,3-butadiényle | H |
| 154 | (3c) | H | H | n-butyle | H |
| 155 | (3c) | H | H | 1-butényle | (9h) |
| 156 | (3c) | H | H | 1-butényle | (9i) |
| 157 | (3c) | OH | H | 1,3-butadiényle | (9a) |
| 158 | (3c) | OH | CH₃ | 1-butényle | (9a) |
| 159 | (3c) | OH | H | 1-propényle | (9a) |

6. Composé de formule (10) :

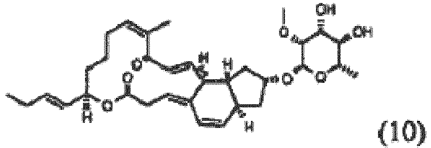

7. Composé de formule (11)

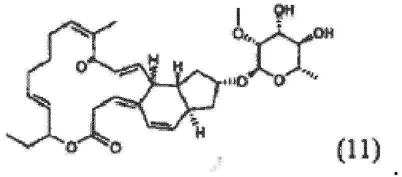

8. Composé selon la revendication 1, dans lequel R⁹ est différent de H.

9. Composé selon la revendication 3, dans lequel R⁹ est différent de H.

10. Composition insecticide ou acaricide, comprenant une quantité, inactivant un insecte, un acarien ou une tique, d'un composé selon la revendication 8, en association avec une matière de support phytologiquement ou physiologiquement acceptable.

11. Composé selon la revendication 8, pour utilisation en tant qu'insecticide ou acaricide.

12. Composé selon la revendication 8, pour utilisation pour la protection d'un lieu contre l'infestation par des insectes, des acariens ou des tiques.

13. Composé selon la revendication 8, pour utilisation pour la lutte contre une population de parasites qui infestent un animal hôte.

14. Composition destinée à être utilisée dans la lutte contre une infestation par des poux chez un sujet humain, comprenant comme composant actif un composé selon la revendication 8, ou un sel physiologiquement acceptable d'un tel composé, et un véhicule physiologiquement acceptable.

15. Composé selon la revendication 8, pour utilisation dans la lutte contre une infestation par des poux chez un sujet humain.

16. Procédé pour la préparation d'un composé selon la revendication 1, comprenant la culture d'une souche choisie parmi
NRRL 30424,
NRRL 30423,
NRRL 30422,
NRRL 30438,
NRRL 30421 et
NRRL 30437,
ou d'une souche dérivée de telles souches, qui produit un composé selon la revendication 1, dans un milieu de culture convenable, dans des conditions de fermentation aérobie submergée, jusqu'à ce que soit produite une quantité récupérable d'un composé selon la revendication 1, et la récupération d'un composé selon la revendication 1 à partir du milieu de culture.

17. Culture biologiquement pure de NRRL 30424 ou souche dérivée de celle-ci, produisant un composé selon la revendication 1.

18. Culture biologiquement pure de NRRL 30423 ou souche dérivée de celle-ci, produisant un composé selon la revendication 1.

19. Culture biologiquement pure de NRRL 30422 ou souche dérivée de celle-ci, produisant un composé selon la revendication 1.

20. Culture biologiquement pure de NRRL 30438 ou souche dérivée de celle-ci, produisant un composé selon la revendication 1.

21. Culture biologiquement pure de NRRL 30421 ou souche dérivée de celle-ci, produisant un composé selon la revendication 1.

22. Culture biologiquement pure de NRRL 30437 ou souche dérivée de celle-ci, produisant un composé selon la revendication 1.
